# EUROPEAN PATENT SPECIFICATION

(11) **EP 4 358 902 B1**
(45) Date of publication and mention of the grant of the patent: **05.08.2026**
(21) Application number: 22828961.7
(22) Date of filing: 04.05.2022
(51) Int. Cl.: A61F 2/44, A61B 17/68, A61B 17/70, A61B 17/80, A61B 17/88, A61F 2/46

(54) **EXPANDABLE INTERBODY IMPLANT AND CORRESPONDING SURGICAL TOOL**
EXPANDIERBARES ZWISCHENWIRBELIMPLANTAT UND ZUGEHÖRIGES CHIRURGISCHES WERKZEUG
IMPLANT INTERVERTÉBRAL EXTENSIBLE ET OUTIL CHIRURGICAL CORRESPONDANT

(30) Priority: 24.06.2021 US 202117356950; 01.11.2021 US 202117515709; 04.02.2022 US 202217665449
(43) Date of publication of application: 01.05.2024
(73) Proprietor: Warsaw Orthopedic, Inc., Warsaw, IN 46581 (US)
(72) Inventor: HAYES, Stanley, Kyle, Mission Viejo, CA 92691 (US); LOKE, Robert, M., Memphis, TN 38103 (US); BARFIELD, Charles, J., East Hernando, MS 38632 (US); PROTOPSALTIS, Dimitri, K., Memphis, TN 38103 (US)
(74) Representative: Viering, Jentschura & Partner mbB Patent- und Rechtsanwälte
(86) International application number: PCT/US2022/027695
(87) International publication number: WO 2022/271280

(56) References cited:
- WO-A1-2018/049227
- WO-A1-2021/055323
- KR-B1- 102 117 224
- US-A1- 2016 100 951
- US-A1- 2017 112 630
- US-A1- 2018 303 621
- US-A1- 2019 133 788
- US-A1- 2021 077 271
- US-B1- 6 190 414

## Description

### FIELD

In one aspect, the present technology is generally related to an externally driven expandable interbody implant for use in a medical procedure related to the spine. In some embodiments, disclosed implants may be used in an anterior cervical discectomy and fusion (ACDF) procedure although other uses in other areas of the spine or between two bones are also contemplated.

### BACKGROUND

Mechanically operated interbody implants may be used to align and/or realign a patient's spine during a medical procedure and/or for purposes of fusion, degenerative tissue and/or trauma/repair procedures. Conventional implants designed for the Thoracic and Lumbar region of the spine often include top and bottom endplates and a mechanical means to separate the top and bottom endplates. The mechanical mechanisms to separate the top and bottom endplates are often cumbersome and require a large footprint that is often unsuitable, for example, for ACDF type surgeries of the cervical portion of the spine. Many currently available ACDF type implants may be limited in an ability to optimize the adjustment of lordosis or sagittal alignment of the vertebral bodies because they may rely on a fixed lordotic angle between the superior/cephalad and inferior/caudad faces of the device. WO 2021/055323 A1, US 2016/100951 A1 and WO 2018/049227 A1 disclose examples of expandable spinal cages that are hinged and can be locked with a locking screw.

### SUMMARY

The techniques of this disclosure generally relate to an expandable interbody implant including a superior endplate and an inferior endplate hingedly coupled and which may further include a locking element to secure the inferior endplate and superior endplate in a particular configuration, for example. The superior and inferior endplates may be moved in a multitude of expanded and/or lordosed or kyphosed or otherwise angled configurations via an external inserter for example. In various embodiments, a locking screw may be a breakoff type screw. In various embodiments at least one breakoff tang on the implant may be used to for gripping of the implant to insert it into a disc space and afterwards the breakoff tang may be broken off and removed. Additionally, in various embodiments the locking screw may be used to grip the implant and insert it into a disc space. Additionally, in various embodiments female recesses, rather than tangs (or male bosses / protrusions), may be used for gripping of the implant and inserting the implant into a disc space.

The present invention relates to an expandable implant according to claim 1 and system with a corresponding surgical tool according to claim 7. Preferred embodiments of the invention are set forth in the dependent claims. In one aspect, the present disclosure provides for an expandable implant movable between a contracted position (closed position) and an expanded position, for example. The expandable implant includes an expandable body extending from a proximal end to a distal end in a proximal-to-distal direction (may also be referred to as an anterior-to-posterior direction depending on surgical technique),extending from a first lateral side to a second lateral side in a widthwise direction, and extend from a superior endplate to an inferior endplate in a heightwise direction (may also be referred to as a cephalad-to-caudal and/or vertical direction depending on surgical technique). In various embodiments, the expandable body is defined by a superior endplate and an inferior endplate that are hingedly connected, for example. In various embodiments, the superior endplate includes a first core having a distal engagement surface (may also be referred to as a posterior engagement surface depending on surgical technique) and the inferior endplate includes a second core having a proximal engagement surface (may also be referred to as an anterior engagement surface) and a threaded screw aperture, for example. In various embodiments, disclosed implants may include a threaded breakoff screw having a fracture surface that is disposed in the threaded screw aperture and movable between a locked position and an unlocked position, for example. In various embodiments, when in the locked position, the breakoff screw urges the distal engagement surface of the first core into direct contact with the proximal engagement surface of the second core, for example.

In another aspect, the disclosure provides for a system including a medical implant and a surgical tool, for example. The system may include an expandable implant movable between a contracted position and an expanded position, for example. In various embodiments, the expanded position may also refer to a distracted and angled orientation of the superior endplate and inferior endplate. The expandable implant may include an expandable body extending from a proximal end to a distal end in a proximal-to-distal direction and extending from a first lateral side to a second lateral side in a widthwise direction, for example. In various embodiments, the expandable body may be defined by a superior endplate and an inferior endplate that are hingedly connected, for example. In various embodiments, the superior endplate includes a first core having a distal engagement surface and the inferior endplate includes a second core having a proximal engagement surface and a threaded screw aperture, for example. In various embodiments, disclosed implants may include a locking screw that is disposed in the threaded screw aperture and movable between a locked position and an unlocked position, for example. In various embodiments, when in the locked position, the locking screw urges the distal engagement surface of the first core into direct contact with the proximal engagement surface of the second core, for example. The system may also include a surgical tool for expanding the implant and tightening the locking screw while the implant is expanded at a desired height, position, and/or angle.

In an aspect, an expandable implant movable between a contracted position and an expanded position, is disclosed. The expandable body may extend from a proximal end to a distal end in a proximal-to-distal direction and extending from a first lateral side to a second lateral side in a widthwise direction, the expandable body being defined by a superior endplate and an inferior endplate that are hingedly connected, for example.

The superior endplate includes a first core having a screw slot and a distal engagement surface. The inferior endplate includes a second core having a proximal engagement surface. The implant includes a locking screw extendable through the first core and second core and movable between a locked position and an unlocked position. In the locked position, the locking screw urges the distal engagement surface of the first core against the proximal engagement surface of the second core.

In another aspect, the superior endplate includes a first gripping indentation located at a proximal end thereof; and the inferior endplate includes a second gripping indentation located at a proximal end thereof, for example.

In another aspect, the first gripping indentation includes a slot having a superior curved surface and an inferior curved surface; and the second gripping indentation includes a slot having a superior curved surface and an inferior curved surface, for example.

According to the invention, the superior endplate includes a first plurality of engagement features that are angled about 20 degrees to about 40 degrees with respect to a proximal surface of the superior endplate; and the inferior endplate includes a second plurality of engagement features that are angled about 20 degrees to about 40 degrees with respect to a proximal surface of the inferior endplate, for example.

According to the invention, the superior endplate further includes a channel located adjacent the distal end and extending in the widthwise direction; and the inferior endplate further includes a rail located adjacent the distal end and extending in the widthwise direction, for example. In various embodiments the rail has a size and shape generally corresponding to a size and shape of the channel and being located within the channel, for example.

In various embodiments, the distal engagement surface of the first core includes a first curved surface; the proximal engagement surface of the second core includes a second curved surface; and the first curved surface is defined by a radius of a circle having a center point that is offset from an axis of rotation of the rail, for example.

According to the invention, the superior endplate includes at least one hook portion adjacent the channel; the inferior endplate includes at least one relief portion adjacent the rail; the at least one hook portion has a size and shape generally corresponding to a size and shape of the at least one relief portion; and the at least one hook portion is disposed within the at least one relief portion, for example.

In various embodiments, in a cross-section view, the channel includes an arcuate shape and the rail includes an arcuate shape, for example.

In various embodiments, the locking screw includes a threaded end and a drive feature separated by a fracture surface for separating the locking screw; the first core is disposed proximally with respect to the second core; and the locking screw is threadably engaged with at least one of the first core and second core, for example.

In various embodiments, the locking screw extends in a proximal to distal direction along a longitudinal axis and may be broken into a proximal portion and a distal portion at a fracture surface; and when broken, the fracture surface is recessed relative to a sidewall of the locking screw, for example.

In another aspect, a system including a medical implant and a surgical tool is disclosed. The system may include an expandable implant movable between a contracted position and an expanded position, for example. In various embodiments, an expandable body extending from a proximal end to a distal end in a proximal-to-distal direction and extending from a first lateral side to a second lateral side in a widthwise direction, the expandable body being defined by a superior endplate and an inferior endplate that are hingedly connected, for example. In various embodiments, the superior endplate includes a first core having screw slot and a distal engagement surface; and the inferior endplate includes a second core having a proximal engagement surface, for example. In various embodiments, a locking screw may be movable between a locked position and an unlocked position, for example. In some embodiments, in the locked position, the locking screw urges the distal engagement surface of the first core against the proximal engagement surface of the second core, for example. In various embodiments, the system may include a surgical tool for moving the implant from a contracted position to an expanded position and for moving the locking screw between the locked position and the unlocked position, for example. In some embodiments, the surgical tool may be capable of moving the locking screw into the locked position while supporting the implant in the expanded position.

In various embodiments, the superior endplate includes a first gripping indentation located at a proximal end thereof; and the inferior endplate includes a second gripping indentation located at a proximal end thereof, for example. In some embodiments, the surgical tool includes a first gripping protrusion having a size and shape generally corresponding to a size and shape of the first gripping indentation; and the surgical tool includes a second gripping protrusion having a size and shape generally corresponding to a size and shape of the second gripping indentation, for example.

In various embodiments, the first gripping indentation includes a slot having a superior curved surface and an inferior curved surface; and the second gripping indentation includes a slot having a superior curved surface and an inferior curved surface, for example.

In various embodiments, the surgical tool includes a pivotal linkage assembly including a first arm and a second arm, for example.

In various embodiments, the surgical tool includes a body portion, a handle, and a third arm, the third arm being fixed relative to the body portion, for example.

In various embodiments, the surgical tool further includes: a body portion having an aperture extending therethrough; a pivotal linkage assembly including a first arm and a second arm; and a third arm that is fixed relative to the body portion, for example. In some embodiments, an outer shaft may extend through the body portion and having a drive feature at a distal end thereof for rotating the locking screw from the unlocked position to the locked position; and an inner shaft may extend through the outer shaft and having a first thread pattern at an end thereof for drawing the expandable implant towards the surgical tool, for example.

In various embodiments, the locking screw includes a recessed fracture surface for separating the locking screw into a proximal portion and a distal portion, and the surgical tool is configured to separate the locking screw into the proximal portion and distal portion while the implant is in the expanded position and retain the proximal portion of the locking screw via the inner shaft, for example.

In various embodiments, the surgical tool further includes an actuator for causing the pivotal linkage assembly to pivot relative to the third arm and thereby expand the implant, for example

In various embodiments, the superior endplate further includes a channel located adjacent the distal end and extending in the widthwise direction; and the inferior endplate further includes a rail located adjacent the distal end and extending in the widthwise direction, the rail having a size and shape generally corresponding to a size and shape of the channel and being located within the channel, for example.

In various embodiments, the distal engagement surface of the first core includes a first curved surface; the proximal engagement surface of the second core includes a second curved surface; and the first curved surface is defined by a radius of a circle having a center point that is offset from an axis of rotation of the rail, for example.

The details of one or more aspects of the disclosure are set forth in the accompanying drawings and the description below. Other features, objects, and advantages of the techniques described in this disclosure will be apparent from the description and drawings, and from the claims.

### BRIEF DESCRIPTION OF DRAWINGS

FIG. 1 is a perspective view of an expandable implant.
FIG. 2 is an alternate perspective view of an expandable implant.
FIG. 3 is a top down view of an expandable implant.
FIG. 4 is a side view of an expandable implant.
FIG. 5 is a rear perspective view of an expandable implant.
FIG. 6 is a perspective view of the interior of a superior endplate of an expandable implant.
FIG. 7 is a perspective view of the interior of an inferior endplate of an expandable implant.
FIG. 8 is a perspective exploded parts view of an expandable implant.
FIG. 9 an exploded parts view of an expandable implant from a side view perspective.
FIG. 10 is a perspective cross section view of an expandable implant.
FIG. 11 is a cross section view of an expandable implant.
FIG. 12 is a side view of a superior endplate for use with at least some expandable implant embodiments.
FIG. 13A is a perspective view of a first expandable implant.
FIG. 13B is a perspective view of a second expandable implant.
FIG. 14A is a perspective view of a third expandable implant.
FIG. 14B is a perspective view of a fourth expandable implant.
FIG. 15 is a perspective view of a fifth expandable implant.
FIG. 16 is a side view of an expandable implant in the expanded configuration.
FIG. 17 is a side view of an expandable implant showing a bone screw trajectory.
FIG. 18 is a front view of an expandable implant.
FIG. 19 is a front view of an enlarged area of FIG. 18.
FIG. 20 is a perspective view of an inserter for use with disclosed expandable implants.
FIG. 21 is a perspective view of an inserter for use with disclosed expandable implants shown in skeleton outlining for ease of understanding.
FIG. 22 is a perspective view of an inserter for use with disclosed expandable implants shown in skeleton outlining for ease of understanding.
FIG. 23A is a rear view of an inserter in a non-expanded position.
FIG. 23B is a rear view of an inserter in an expanded position.
FIG. 24 is an enlarged view of a distal end of an inserter in an expanded position coupled to an example expandable implant in a corresponding expanded position.
FIG. 25 is a perspective view of an expandable implant in an expanded configuration after a breakoff portion of a locking screw has been broken off.
FIG. 26 is a perspective view of a surgical instrument for use with disclosed expandable implants.
FIG. 27 is a perspective view of a surgical instrument for use with disclosed expandable implants.
FIG. 28 is a perspective view of an expandable implant after the mounting tangs have been broken off.
FIG. 29 is a reference drawing showing the human spine of which various disclosed implant embodiments may be installed in.
FIG. 30 is a reference drawing showing various planes and reference directions of which the various disclosed implant embodiments may move in or act in with respect to a patient.
FIG. 31 is a perspective view of a second implant embodiment.
FIG. 32 is a first perspective exploded parts view of the second implant embodiment.
FIG. 33 is a second perspective exploded parts view of the second implant embodiment.
FIG. 34 is a side exploded parts view of the second implant embodiment.
FIG. 35 is a first side view of a breakoff screw having a recessed fracture surface.
FIG. 36 is a second side view of a breakoff screw having a recessed fracture surface.
FIG. 37 is a cross section view of a breakoff screw having a recessed fracture surface.
FIG. 38 is a perspective view of a swaging fixture.
FIG. 39 is a cross section view of a swage mandrel and a distal end of a breakoff screw before the commencement of a swage process.
FIG. 40 is a cross section view showing a result of a swage process.
FIG. 41 is an enlarged view of region S-W of FIG. 40.
FIG. 42A is a front perspective view of a third implant embodiment.
FIG. 42B is an alternate front perspective view of a third implant embodiment.
FIG. 43A is a front perspective view of an implant having angled engagement features.
FIG. 43B is a top down view of an implant having angled engagement features.
FIG. 44 is a perspective view of a surgical tool for use with disclosed implant embodiments.
FIG. 45 is a first side view of a surgical tool for use with disclosed implant embodiments.
FIG. 46A is a second side view of a surgical tool for use with disclosed implant embodiments.
FIG. 46B is a third side view of a surgical tool in an operative position for use with disclosed implant embodiments.
FIG. 46C is a perspective view of a surgical tool in the operative position.
FIG. 47A is first exploded parts view of a surgical tool for use with disclosed implant embodiments.
FIG. 47B is second exploded parts view of a surgical tool for use with disclosed implant embodiments.
FIG. 48 is a perspective view showing a surgical tool immediately prior to coupling with a surgical implant.
FIG. 49 is a first cross section drawing of the surgical tool and implant in a coupled configuration.
FIG. 50 is a second cross section drawing of the surgical tool and implant in a coupled configuration.
FIG. 51 is a third cross section drawing of the surgical tool and implant in a coupled configuration.
FIG. 52 is a perspective view of an expandable implant.
FIG. 53 is an alternate perspective view of an expandable implant.
FIG. 54 is a top down view of an expandable implant.
FIG. 55 is a perspective exploded parts view of an expandable implant.
FIG. 56 is an alternate perspective exploded parts view of an expandable implant.
FIG. 57 is a side view of a superior endplate.
FIG. 58 is a side view of an expandable implant in a contracted position.
FIG. 59 is a side view of an expandable implant in an expanded position.
FIG. 60 is a rear view of an expandable implant.
FIG. 61 is a front view of an expandable implant with a locking screw.
FIG. 62 is an exploded parts view of a superior endplate, inferior endplate, and core partially rotated for viewing of various engagement surfaces.
FIG. 63 is a reference drawing showing the human spine of which various disclosed implant embodiments may be installed in.
FIG. 64 is a reference drawing showing various planes and reference directions of which the various disclosed implant embodiments may move in or act in with respect to a patient.

### DETAILED DESCRIPTION

Embodiments of the present disclosure relate generally, for example, to spinal stabilization systems, and more particularly, to surgical instruments for use with spinal stabilization systems. Embodiments of the devices and methods are described below with reference to the Figures. While the implantation methods described herein do not form part of the invention, they are disclosed as they represent useful background for understanding the invention.

The following discussion omits or only briefly describes certain components, features and functionality related to medical implants, installation tools, and associated surgical techniques, which are apparent to those of ordinary skill in the art. It is noted that various embodiments are described in detail with reference to the drawings, in which like reference numerals represent like parts and assemblies throughout the several views, where possible. Reference to various embodiments does not limit the scope of the claims appended hereto because the embodiments are examples of the inventive concepts described herein. Additionally, any example(s) set forth in this specification are intended to be non-limiting and set forth some of the many possible embodiments applicable to the appended claims. Further, particular features described herein can be used in combination with other described features in each of the various possible combinations and permutations unless the context or other statements clearly indicate otherwise.

Terms such as "same," "equal," "planar," "coplanar," "parallel," "perpendicular," etc. as used herein are intended to encompass a meaning of exactly the same while also including variations that may occur, for example, due to manufacturing processes. The term "substantially" may be used herein to emphasize this meaning, particularly when the described embodiment has the same or nearly the same functionality or characteristic, unless the context or other statements clearly indicate otherwise.

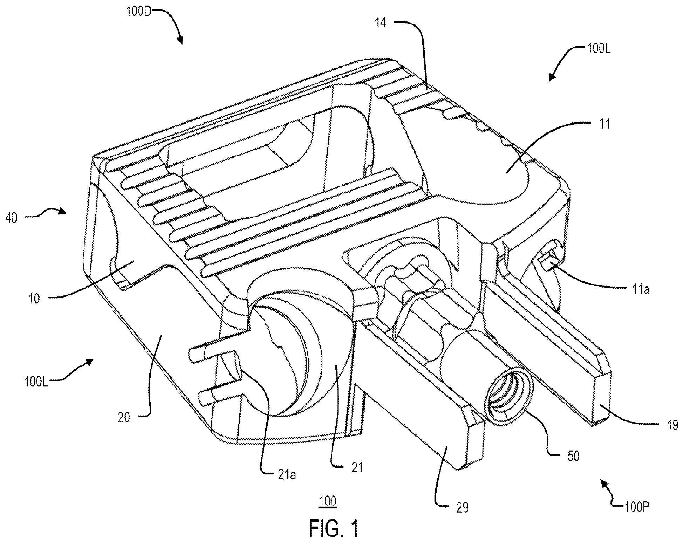

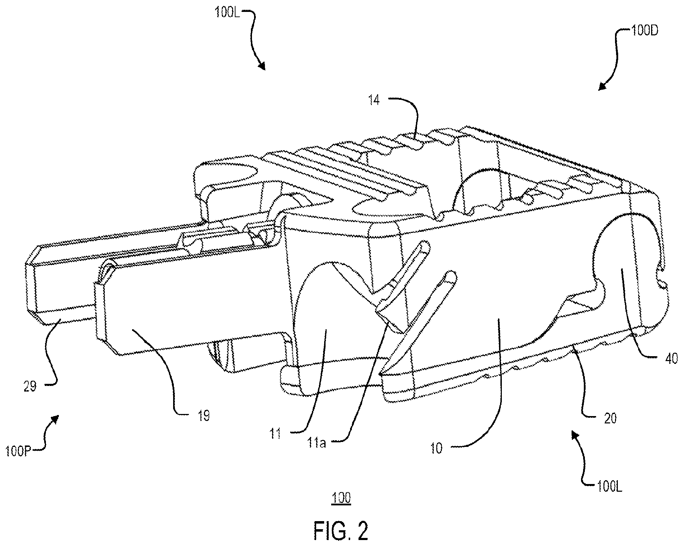

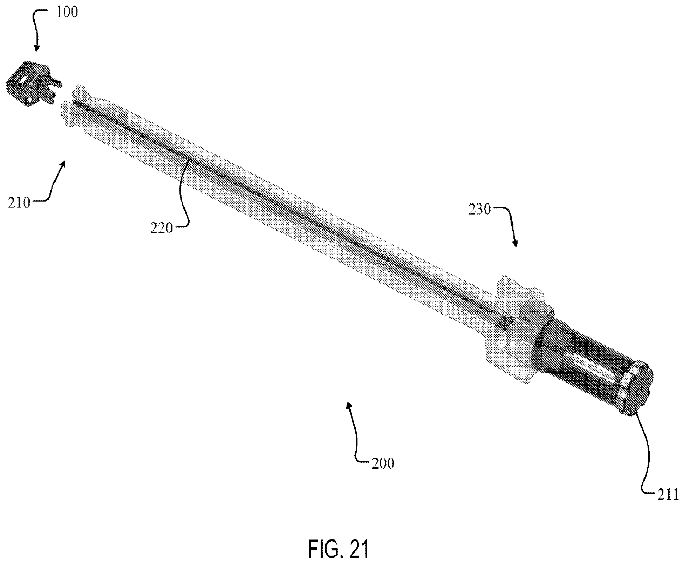

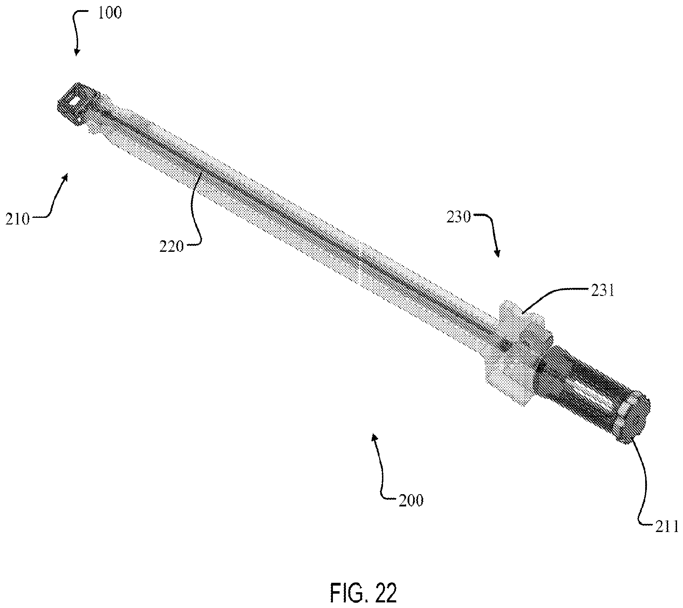

Referring to FIGS. 1-43 generally, various embodiments and views of an expandable implant 100 are disclosed. The components of expandable implant 100 can be fabricated from biologically acceptable materials suitable for medical applications, including metals, synthetic polymers, ceramics and bone material and/or their composites. For example, the components, individually or collectively, can be fabricated from materials such as stainless steel alloys, commercially pure titanium, titanium alloys, Grade 5 titanium, super-elastic titanium alloys, cobalt-chrome alloys, superelastic metallic alloys (e.g., Nitinol, super elasto-plastic metals, such as GUM METAL^{™}), ceramics and composites thereof such as calcium phosphate (e.g., SKELITE^{™}), thermoplastics such as polyaryletherketone (PAEK) including polyetheretherketone (PEEK), polyetherketoneketone (PEKK) and polyetherketone (PEK), carbon-PEEK composites, PEEK-BaSO4 polymeric rubbers, polyethylene terephthalate (PET), fabric, silicone, polyurethane, silicone-polyurethane copolymers, polymeric rubbers, polyolefin rubbers, hydrogels, semi-rigid and rigid materials, elastomers, rubbers, thermoplastic elastomers, thermoset elastomers, elastomeric composites, rigid polymers including polyphenylene, polyamide, polyimide, polyetherimide, polyethylene, epoxy, bone material including autograft, allograft, xenograft or transgenic cortical and/or corticocancellous bone, and tissue growth or differentiation factors, partially resorbable materials, such as, for example, composites of metals and calcium-based ceramics, composites of PEEK and calcium based ceramics, composites of PEEK with resorbable polymers, totally resorbable materials, such as, for example, calcium based ceramics such as calcium phosphate, tri-calcium phosphate (TCP), hydroxyapatite (HA)-TCP, calcium sulfate, or other resorbable polymers such as polyaetide, polyglycolide, polytyrosine carbonate, polycaroplaetohe, polylactic acid or polylactide and their combinations.

In various embodiments, components may be coated with a ceramic, titanium, and/or other biocompatible material to provide surface texturing at (a) the macro scale, (b) the micro scale, and/or (c) the nano scale, for example. Similarly, components may undergo a subtractive manufacturing process providing for surface texturing configured to facilitate osseointegration and cellular attachment and osteoblast maturation. Example surface texturing of additive and subtractive manufacturing processes may comprise (a) macro-scale structural features having a maximum peak-to-valley height of about 40 microns to about 500 microns, (b) micro-scale structural features having a maximum peak-to-valley height of about 2 microns to about 40 microns, and/or (c) nano-scale structural features having a maximum peak-to-valley height of about 0.05 microns to about 5 microns. In various embodiments, the three types of structural features may be overlapping with one another, for example. Additionally, such surface texturing may be applied to any surface, e.g., both external exposed facing surfaces of components and internal non exposed surfaces of components. Further discussion regarding relevant surface texturing and coatings is described in, for example, U.S. Pat. No. 11,096,796, titled Interbody spinal implant having a roughened surface topography on one or more internal surfaces, and filed on March, 4, 2013. Accordingly, it shall be understood that any of the described coating and texturing processes of U.S. Pat. No. 11,096,796, may be applied to any component of the various embodiments disclosed herein, e.g., the exposed surfaces and internal surfaces of endplates. Another example technique for manufacturing an orthopedic implant having surfaces with osteoinducting roughness features including micro-scale structures and nano-scale structures is disclosed in U.S. Pat. No. 10,821, 000. Additionally, an example of a commercially available product may be the Adaptix^{™} Interbody System sold by Medtronic Spine and comprising a titanium cage made with Titan nanoLOCK^{™}.

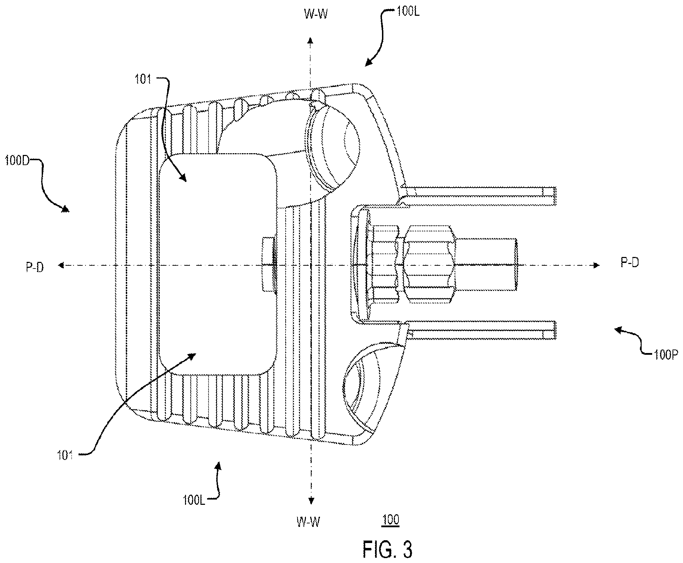

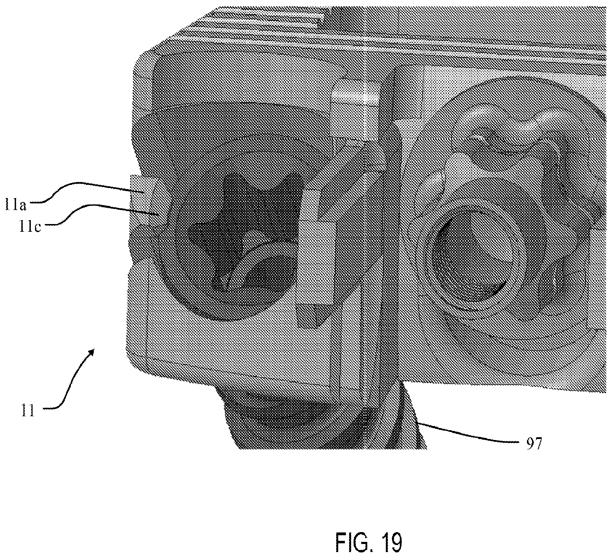

Referring generally to FIGS. 1-5 various views of an expandable implant 100 in a collapsed position are illustrated. FIGS. 1-2 are various perspective views of an expandable implant 100. FIG. 3 is a top down view of an expandable implant 100. In the example embodiment, expandable implant 100 may include a proximal end 100P, a distal end 100D, and first and second lateral sides 100L. Additionally, a pair of bone screw apertures 11, 21 may be positioned on the proximal end 100P, for example. In various embodiments, bone screw apertures 11, 21 may comprise a corresponding bone screw retention mechanism 11a, 21a (may also be referred to as an anti-backout locking mechanism). In the example embodiment, the bone screw retention mechanisms 11a, 21a, comprise a flexible tang member having a hook portion at an end thereof that allows the flexible tang member to flex outward in a lateral direction away from the corresponding bone screw aperture 11, 21 during initial installation of the bone screw and to flex back inward towards the corresponding bone screw aperture 11, 21 to prevent a corresponding bone screw from backing out. For example, as the bone is installed in bone screw aperture 11, 21, the bone screw retention mechanism 11a, 21a, may flex outward as the underside of the head portion contacts inclined surface 11c (see FIG. 19).

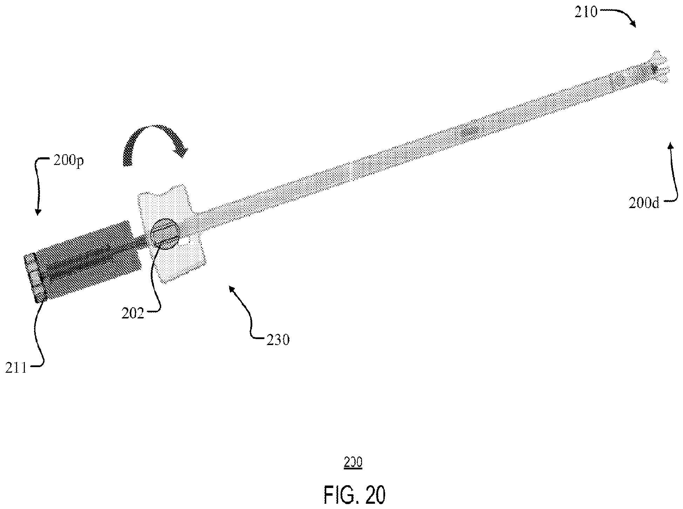

In various embodiments, and as illustrated in FIGS. 1-2, mounting tangs 19, 29 may extend in a proximal direction (may also be referred to as an anterior direction depending on surgical technique and orientation), for example. In various embodiments, implant 100 may be referred to as an externally driven expandable implant because an end user or surgeon may use a surgical tool to open and close implant 100, e.g. expand implant 100. For example, an external tool may adjust the lordotic angle of implant 100 as will be explained in detail with respect to FIGS. 20-25. Once implant 100 is expanded to an appropriate lordotic angle (also referred to as angle of inclination), an end user may fix the relative angle of the superior endplate 10 relative to the inferior endplate 20 by tightening locking screw 50, for example. In some embodiments, superior endplate 10 may be referred to as a "cephalad" endplate and inferior endplate 20 may be referred to as a "caudal" endplate.

Locking screw 50 may also be used in other embodiments, such as fixation of posterior rods, fixation of pedicle screws, and other set screw constructs. Additionally, locking screw 50 may be referred to as a "breakoff screw" in some embodiments.

At least one advantage of relying on an external tool to adjust a lordotic angle of implant 100 may be the reduction of internal components within implant 100 relative to other forms of implants relying on various moving mechanisms and/or expansion mechanisms, for example. Accordingly, in various embodiments, implant 100 may have a relatively large void space in the interior thereof, which may facilitate a fusion process during an ACDF procedure. For example, implant 100 may have a relatively large internal volume 101 that is open through the superior endplate 10 and inferior endplate 20 which may be packed with bone graft material, for example.

As illustrated in FIG. 3, implant 100 may extend in a proximal-to-distal direction (may also referred to as a longitudinal direction and/or an anterior-to-posterior direction depending on surgical technique and final orientation) from the proximal end 100P (may be referred to as anterior end depending on surgical technique and final orientation) to the distal end 100D (may be referred to as posterior end depending on surgical technique and final orientation) though axis P-D through the center of the implant 100, for example. Implant 100 may extend in a widthwise direction (also referred to as lateral direction) from the first lateral side 100L to the second lateral side 100L through axis W-W through the center of the implant 100 and the center of locking screw 50, for example. The axis P-D may be perpendicular and/or substantially perpendicular to the axis W-W. For example, the proximal-to-distal direction may be perpendicular to the widthwise direction. Additionally, a width of the implant may taper from a proximal end 100P where it is widest towards a distal end 100D where it is narrowest. In various embodiments, implant 100 may extend from a superior endplate 10 to an inferior endplate 20 in a heightwise direction (may also be referred to as a cephalad-to-caudal and/or vertical direction depending on surgical technique and final orientation).

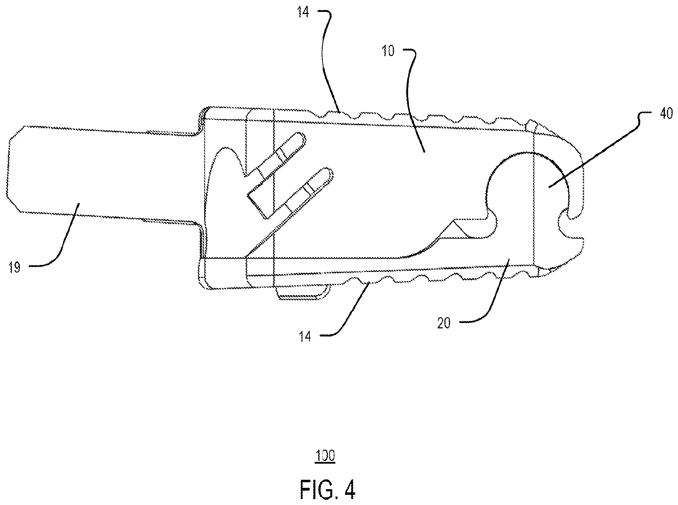

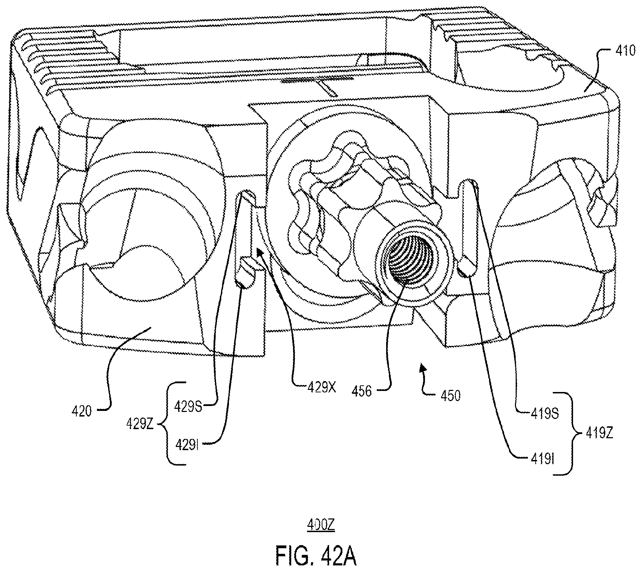

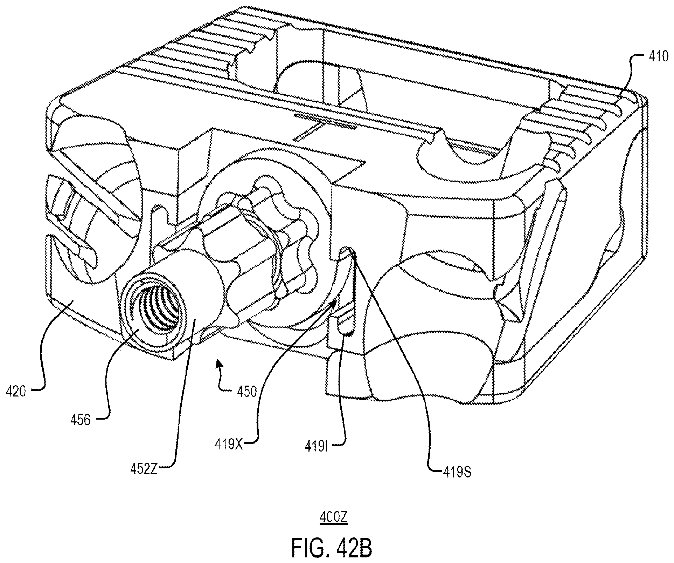

FIG. 4 is a side view of an expandable implant 100. In the example illustration, it is shown that a superior endplate 10 is connected to an inferior endplate 20 such that the superior endplate may pivot about a hinge member 40. In the example embodiment, hinge member 40 comprises an arcuate rail portion of inferior endplate 20 that extends in the widthwise direction, for example. In other embodiments, the hinge member 40 may be reversed relative to the superior and inferior endplates than as illustrated in FIG. 4. In the example embodiment, hinge member 40 may be nested into a corresponding arcuate cavity of the superior endplate 10 such that superior endplate 10 may expand and/or otherwise rotate about hinge member 40. Additionally, in various embodiments the superior endplate 10 and/or inferior endplate 20 may include various engagement elements 14 for engaging with an adjacent boney structure such as a vertebrae, for example. In the example embodiment, the engagement elements comprise a series of alternating rails and valleys therebetween that extend in the widthwise direction. In some embodiments, the valleys may be angled about 20-40 degrees and in at least one embodiment the valleys may be angled at about 30 degrees relative to the corresponding rail (see FIGS. 42A and 42B). At least one advantage of this orientation may be a relatively greater resistance and/or suppression of expulsion of the implant 100 in both the lateral direction and in the proximal-to-distal direction. However, claws, hooks, dimples, spikes, etc. are also contemplated as example engagement elements 14. In some embodiments, an acid etching process may be utilized to form a roughened or textured surface to facilitate securing the implant between boney portions and/or suppressing expulsion of implant 100.

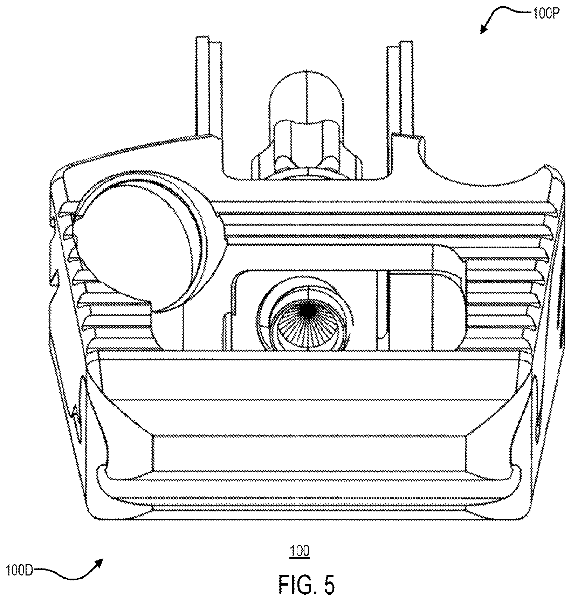

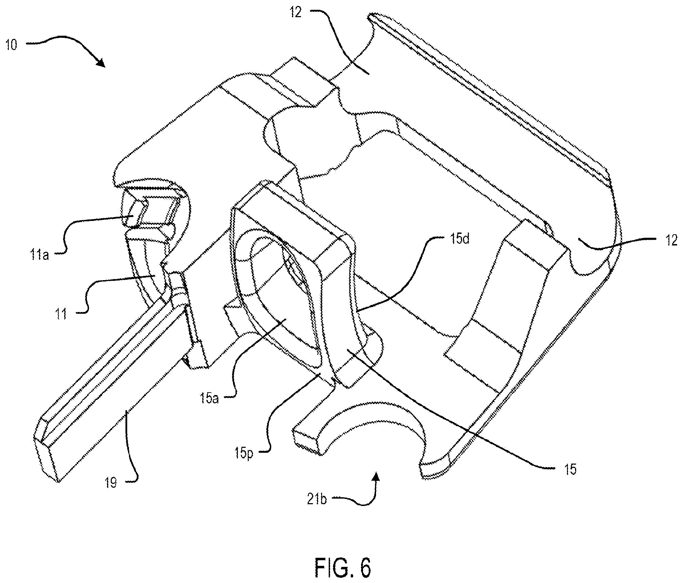

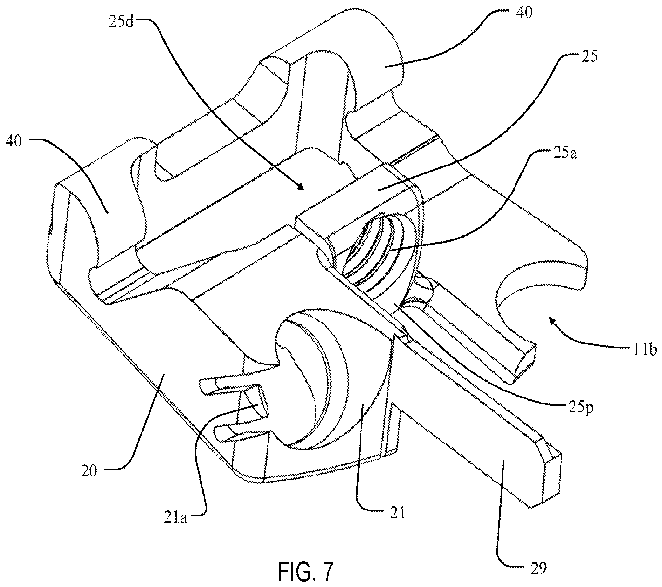

FIG. 5 is a rear perspective view of an expandable implant 100. In the example illustration it is shown that the distal end 100D is narrower than the proximal end 100P. FIG. 6 is a perspective view of the interior of a superior endplate 10. In the example illustration, it is shown that the distal end of superior endplate 10 includes an arcuate channel 12 of which the hinge member 40 may be disposed inside of. The proximal end of superior endplate 10 may include a bone screw aperture cutout 21b to allow a relief area for a corresponding bone screw to be insert through bone screw aperture 21 of inferior endplate 20, for example. Superior endplate 10 may also include a core 15 comprising an aperture 15a that extends from a proximal surface 15p thereof to a distal surface 15d thereof, for example. In various embodiments, aperture 15a may be referred to as a "slot'" or "screw slot". In some embodiments, core 15 may be referred to as a support frame and take a generally rectangular shape. In various embodiments, the distal surface 15d may be curved and generally face the distal end 100D of implant 100. FIG. 7 is a perspective view of the interior of an inferior endplate 20. In the example illustration, it is shown that the distal end of inferior endplate 20 includes a hinge member 40 in the form of an arcuate rail that may be disposed inside of the arcuate channel 12 of the superior endplate 10, for example. The proximal end of inferior endplate 20 may include a bone screw aperture cutout 11b to allow a relief area for a corresponding bone screw to be insert through bone screw aperture 11 of superior endplate 10, for example. Inferior endplate 20 may also include a core 25 comprising a threaded aperture 25a that extends from a proximal surface 25p thereof to a distal surface 25d thereof, for example. In some embodiments, core 25 may be referred to as a support frame and take a generally rectangular shape. In various embodiments, the superior endplate 10 and inferior endplate 20 may each be formed by a unitary single piece, respectively.

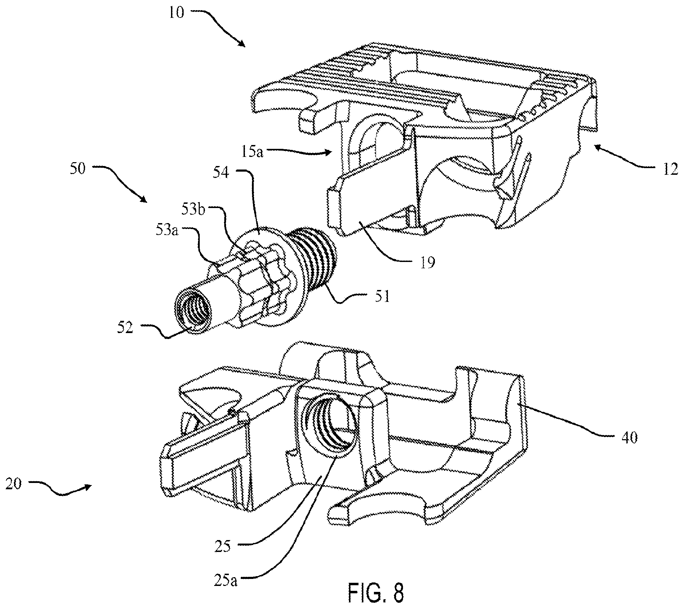

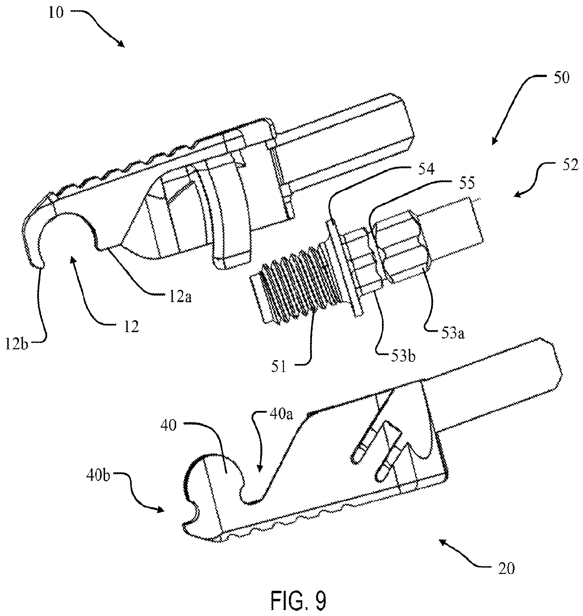

FIG. 8 is a perspective exploded parts view and FIG. 9 is an exploded parts view from a side view perspective of an expandable implant 100. In the example embodiment, a locking screw 50 is illustrated. Locking screw 50 may include an external thread pattern 51 on an outside circumferential surface thereof, for example. The external thread pattern 51 of locking screw 50 may have a size and shape generally corresponding to the threaded aperture 25a of core 25 of inferior endplate 20, for example. In various embodiments, an engagement surface 54 may be disposed adjacent and proximal of external thread pattern 51. In the example embodiment, engagement surface 54 is shaped like a washer and is directly connected to locking screw 50. However, in other embodiments, engagement surface 54 may be a washer or separated element, for example. In some embodiments, engagement surface 54 may be conically shaped and/or spherically shaped. Engagement surface 54 may include a relatively planar and/or flat distal surface and/or proximal surface (anterior / ventral surface). In various embodiments, a proximal end of set screw 50 may include an aperture having an internal threaded surface 52. For example, a cylindrical shaped proximal end may include an aperture having a thread pattern disposed on an internal circumferential surface of the cylindrical shaped proximal end. In the example embodiment, a first drive feature 53a and a second drive feature 53b may be disposed adjacent to and distally with respect to a proximal most end of set screw 50. Additionally, first and second drive features 53a, 53b may be disposed proximally with respect to engagement surface 54. In the example embodiment, drive features 53a, 53b take a hexalobular shape, although various other shapes such as hexagonal, polygonal, Torx, etc. are also contemplated. In some embodiments, a surgical drive tool having a corresponding socket may be coupled to drive features 53a, 53b to cause rotation of locking screw 50. Similarly, in some alternative embodiments, a drive tool with a protruding threaded member having a thread pattern with a corresponding size and shape to internal threaded surface 52 may also cause rotation of set screw 50.

As seen best in FIG. 9, set screw 50 may also include a breakoff location 55, for example. In the example embodiment, breakoff location 55 is disposed directly between drive features 53a, 53b and is designed to shear off when a sufficient rotational force (torque) is applied to a proximal end of set screw 50 while a distal end of set screw 50 is stationary, e.g., when set screw 50 is secured in a locked position and a continued rotational force (torque) is applied to the proximal end of set screw 50 the drive feature 53a and cylindrical end having the internal threaded surface 52 may breakoff. In various embodiments, the internal threaded surface 52 may also be utilized to ensure that the broken off portion is removed from the patient and remains connected to a surgical tool / breakoff tool. As also seen best in FIG. 9, the inferior endplate 20 includes a first relief 40a and a second relief 40b on opposite sides of hinge member 40. The first relief 40a has a size and shape corresponding to a size and shape of a first portion 12a of superior endplate 10 and the second relief 40b has a size and shape corresponding to a size and shape of a second portion 12b of superior endplate 10, for example. In various embodiments, portions 12a, 12b comprises a hook shape, outdent, and/or protrusion, for example. In the example embodiment, portions 12a, 12b may be disposed on opposite sides of channel 12 and cup hinge member 40 such that the superior endplate 10 and inferior endplate 20 may rotate relative to one another without becoming uncoupled.

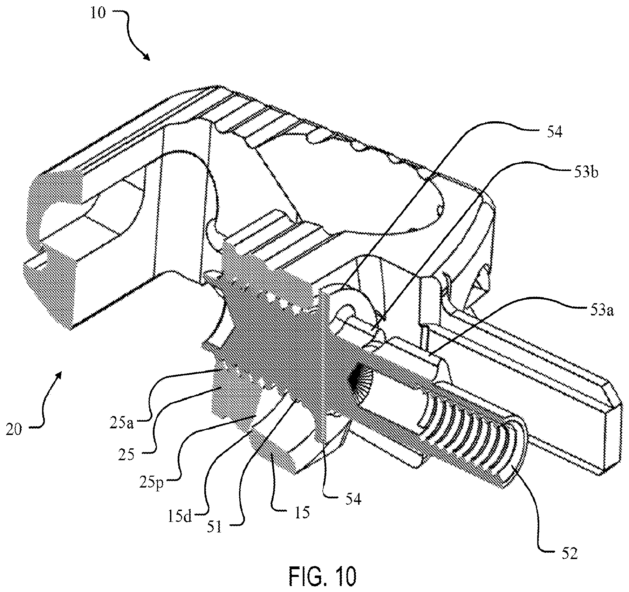

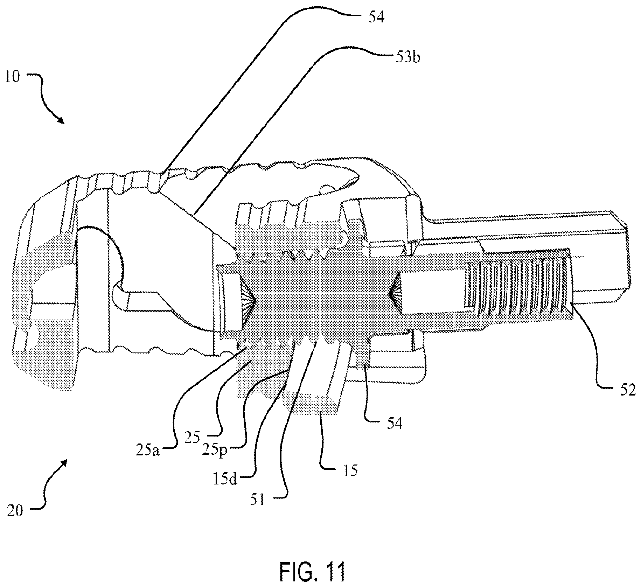

FIG. 10 is a perspective cross section view and FIG. 11 is a cross section view of expandable implant 100. In the example embodiment, the superior endplate 10 and inferior endplate 20 are coupled together by hinge member 40, and core 25 may be positioned behind of core 15, e.g., core 25 may be positioned distally with respect to core 15. Additionally, the outside external thread pattern 51 of locking screw 50 may engage with the threaded aperture 25a of the core 25 and extend through aperture 15a of core 15. In this way, when locking screw 50 is rotated, the distal surface 15d of core 15 may engage with the proximal surface 25p of core 25. For example, by tightening locking screw 50 the engagement surface 54 of locking screw 50 pushes against the proximal surface 15p of core 15 thereby bringing the superior endplate 10 and inferior endplate 20 into frictional engagement.

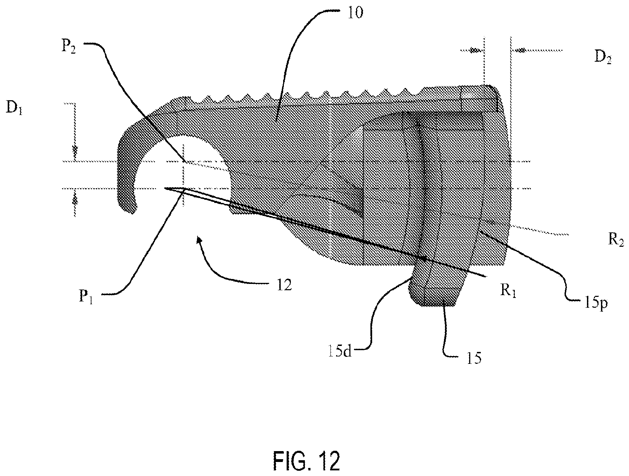

FIG. 12 is a side view of a superior endplate 10 for use with at least some expandable implant 100 embodiments. In the example embodiment, superior endplate 10 may include an arcuate channel 12 of which the hinge member 40 may be disposed inside of. In various embodiments, arcuate channel 12 may be defined by a first circle having a center point at P₁ and/or a segment of the circle having the center point at P₁. The center point P₁ may define an axis of rotation that superior endplate 10 may rotate and/or pivot with respect to inferior endplate 20. For example, superior endplate 10 may be hingedly coupled to hinge member 40 as explained above and rotatable about an axis of rotation defined by center point P₁, for example. Additionally, in various embodiments a distal surface 15d of core 15 may be a curved surface defined (in part or in total) by a second circle having a center point at P₁ and a radius R₁. The proximal surface 15p of core 15 may also be a curved surface defined (in part or in total) by a segment of a circle having a radius R₂ and a center point P₂. In the example embodiment, P₂ is located a distance D₁ above point P₁ and radius R₂ is greater than radius R₁. Additionally, the proximal surface 15p of core 15 is offset a distance D₂ from the proximal most face of the superior endplate 10. In the example embodiment, center point P₂ is vertically above center point P₁ however, in other embodiments, center point P₂ may be offset by a greater amount or even a lesser amount than illustrated. In some examples, P₂ may not be aligned vertically above Pi. In various embodiments, R₁ may be about 7-9 mm +/- about 1 mm and R₂ may be about 8-10 mm +/- about 1 mm although these numbers may be modified in some embodiments having a larger or smaller footprint. In various embodiments D₁ is about 0.25 mm to about 1.0 mm and D₂ is about 0.25 mm to about 1.25 mm. In at least one embodiment, D₁ is about 0.75 mm and D₂ is about 0.8 mm and R₂ is about 9.2 mm.

The above explained geometrical relationship between the offset center points P₁ and P₂ and R₁ and R₂ may have several advantages in terms of operability and functionality. At least one advantage is that the superior endplate 10 may have a natural tendency to apply a force against the engagement surface 54 of locking screw 50 such that locking screw 50 may function similar to a wedge preventing implant 100 from fully collapsing. Another advantage is that a biasing force may be applied that naturally urges the superior endplate 10 and inferior endplate 20 into an expanded position which may assist with expanding the implant 100 when positioned between a superior vertebrae and an inferior vertebrae, for example. For example still, an end user such as a surgeon may expand implant 100 and the offset arrangement explained above may facilitate the function of keeping implant 100 lordosed at the chosen angle.

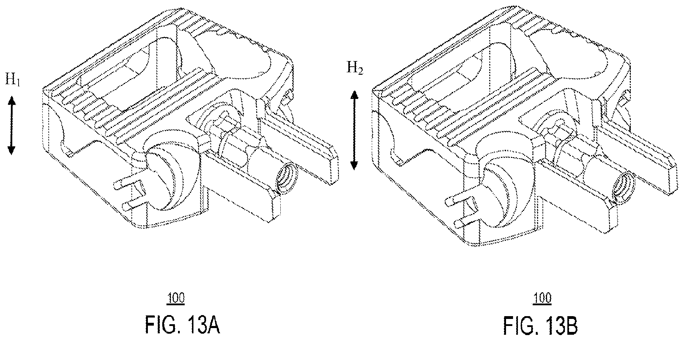

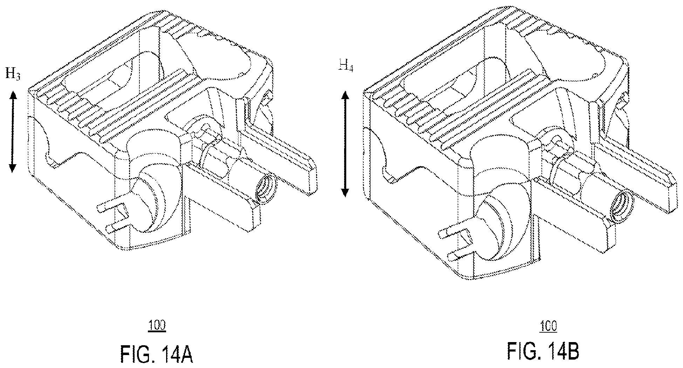

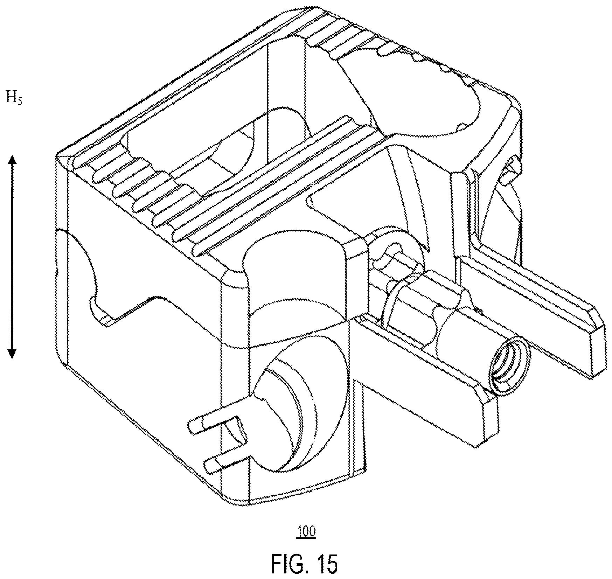

FIG. 13A is a perspective view of a first expandable implant, FIG. 13B is a perspective view of a second expandable implant, FIG. 14A is a perspective view of a third expandable implant, FIG. 14B is a perspective view of a fourth expandable implant, and FIG. 15 is a perspective view of a fifth expandable implant. In the series of illustrations it is shown that various embodiments in accordance with the principles of this disclosure may be variously sized depending on the particular location in a human body and the particular patient specific human anatomy. For example, FIG. 13A illustrates a first expandable implant 100 having a first height H₁ or thickness between the superior endplate 10 and inferior endplate 20, FIG. 13B illustrates a second expandable implant 100 having a second height H₂ or thickness, FIG. 14A illustrates a third expandable implant 100 having a third height H₃ or thickness, FIG. 14B illustrates a fourth expandable implant 100 having a fourth height H₄ or thickness, and FIG. 15 illustrates a fifth expandable implant 100 having a fifth height H₅ or thickness. In at least some embodiments, H₁ may be about 5 mm, H₂ may be about 6 mm, H₃ may be about 7 mm, H₄ may be about 8 mm, H₅ may about 9 mm, for example. In various embodiments, an angle of inclination between the superior endplate 10 and inferior endplate 20 may be about 4 degrees to about 15 degrees in an expanded configuration, e.g., an angled and/or inclined configuration.

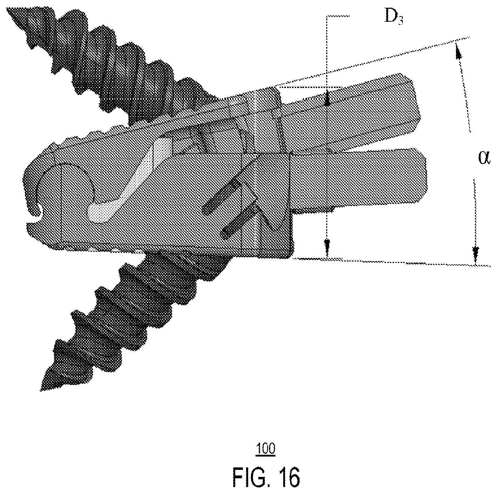

FIG. 16 is a side view of an expandable implant 100 in the expanded configuration. In an expanded position, a distance D₃ between the superior endplate 10 and inferior endplate 20 at the proximal end 100P may be relatively greater than in the closed configuration, for example. Additionally, an angle of inclination α may be relatively greater in an expanded position than in the closed configuration, for example. In this embodiment, implant 100 may have a height H₁ corresponding to FIG. 13A and be about 5 mm in a closed configuration. In the illustrated expanded configuration of FIG. 16, D₃ may be about 8 mm to 9 mm and α may be about 10 degrees to about 20 degrees. In at least one embodiment, D₃ may be 8 mm in a fully expanded position and α may be about 15 degrees, for example.

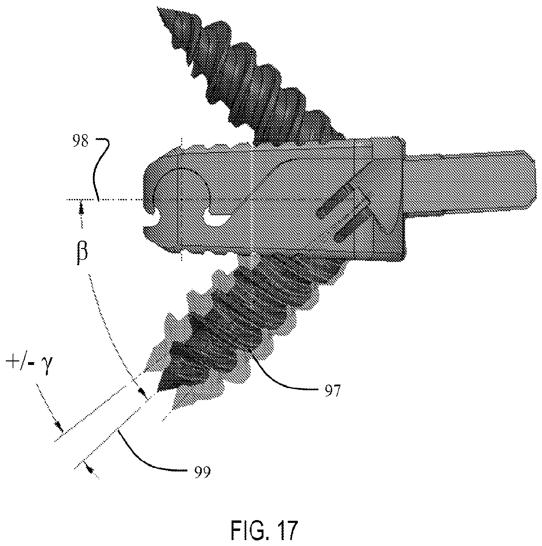

FIG. 17 is a side view of an expandable implant 100 showing a bone screw trajectory 99. In the example embodiment, it is shown that a centered bone screw trajectory 99 of bone screw 97 is at an angle β with respect to a plane 98 that crosses through a center of the implant from a first lateral side to a second lateral side, for example. Additionally, the bone screw trajectory 99 may be varied +/- by a degree y, for example. In various embodiments, β may be about 30 degrees to about 50 degrees and γ may be about 2 degrees to about 10 degrees. In the example embodiment, β may be about 40 degrees and γ may be about 5 degrees.

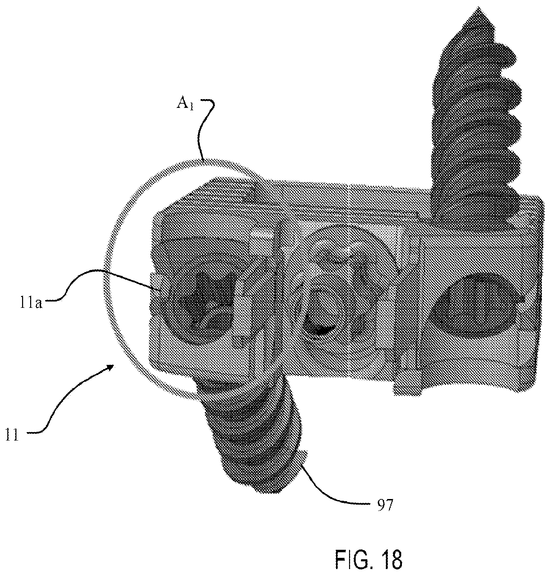

FIG. 18 is a front view of an expandable implant showing an area A₁ and FIG. 19 is a front view of an enlarged area A₁ of FIG. 18. In the example embodiment, bone screw 97 is in a position extending through bone screw aperture 11 where it cannot backout due to bone screw retention mechanism 11a. The bone screw retention mechanism 11a includes an inclined surface 11c such that when bone screw 97 is being installed, an underside of the head portion of bone screw 97 directly contacts the inclined surface 11c thereby pushing the bone screw retention mechanism 11a laterally outward and away from bone screw aperture 11, for example. Thereafter, when bone screw 97 is installed and the head portion of bone screw 97 is beneath inclined surface 11c the bone screw retention mechanism may flex back towards bone screw aperture 11 such that it will prevent bone screw 97 from backing out, e.g., a blocking surface of bone screw retention mechanism 11a may contact an upper surface of the head portion of bone screw 97. In the example embodiment, bone screw retention mechanism 11a comprises flexible arm (or spring tab) having an inclined surface 11c (or ramp) that is disposed on a lateral end of implant 100 adjacent bone screw aperture 11.

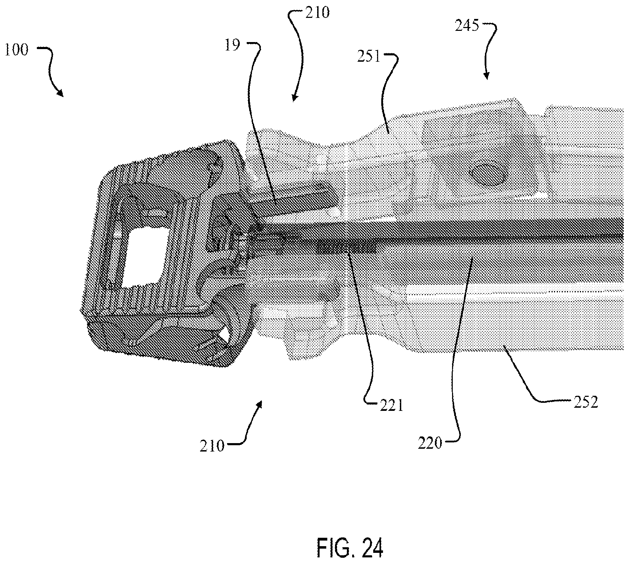

Referring generally to FIGS. 20-24 an inserter 200 for use with disclosed expandable implants 100 is illustrated. Inserter 200 may extend in a longitudinal direction from a proximal end 200p to a distal end 200d, for example. Inserter 200 may include a pair of handles 230, a handle lock 202, and mounting arms 210 for securely coupling to mounting tangs 19, 29 of implant 100, for example. Inserter 200 may further include a tightening knob 211 that is connected to a drive shaft 220 having a drive end 221. Drive end 221 may have a size and shape generally corresponding to a size and shape of the various drive features of locking screw 50, for example the internal threaded surface 52, first drive feature 53a, and/or second drive feature 53b. In the example embodiment shown in FIG. 24, drive end 221 includes an end portion having an outside threaded surface with a corresponding size and shape to the internal threaded surface 52 of locking screw 50. In various embodiments, tightening knob 211 may rotate drive shaft 220 and drive end 221 to engage drive end 221 with locking screw 50 and pull implant 100 towards inserter tool 200 such that mounting tangs 19, 29 are securely nested within corresponding channels of mounting arms 210. Additionally, an end user may rotate inserter 200 thereby translating a rotational force through drive end 221 to locking screw 50, e.g., via first drive feature 53a, and/or second drive feature 53b.

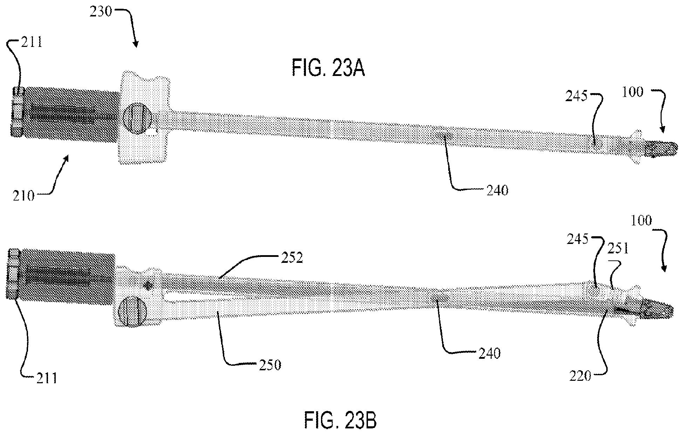

As seen best in FIGS. 23A and 23B, inserter 200 may include a pair of handles 230, a stationary arm 252, a primary pivoting arm 250, and a secondary pivoting arm 251. For example, to expand implant 100 an end user may toggle handle lock 202 to an unlocked position and push down on thumb indentation 231 of the handle 230 that is connected to the primary pivoting arm 250 and secondary pivoting arm 251. In doing so, primary pivoting arm 250 may pivot with respect to medial pivot point 240 and secondary pivoting arm 251 may pivot with respect to distal pivot point 245, for example. The path of travel of secondary pivoting arm 251 may lift up on the corresponding mounting tang 19 or 29 and cause the superior endplate 10 and inferior endplate 20 to separate from one another at the proximal end, for example. In doing so, an end user can lordose implant 100 to a desired angle. For example, as seen best in FIG. 24, secondary pivoting arm 251 has lifted up on mounting tang 19, which is nested in a corresponding channel of mounting arm 210.

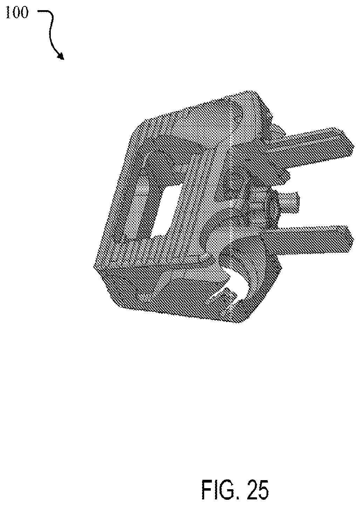

Once implant 100 is lordosed to a desired configuration, an end user may rotate drive shaft 220 and drive end 221 to tighten locking screw 50 as explained previously. After locking screw 50 is relatively tight, the end user may continue to apply a rotational force to locking screw 50 until a proximal portion comprising the cylindrical part having an internal threaded surface 52, and the first drive feature 53a breaks off at breakoff location 55. For example, once locking screw 50 is tightened to a designed torque, the locking screw 50 may shear off as explained previously. At least one advantage of utilizing the locking screw 50, is that it may prevent over tightening which can cause deformation to implant 100. As shown in FIG. 25, implant 100 has been expanded to a desired position and/or lordotic angle. Additionally, locking screw 50 has locked the relative position of the superior endplate 10 with respect to the inferior endplate 20 and the proximal portion of locking screw 50 has been broken off as explained above.

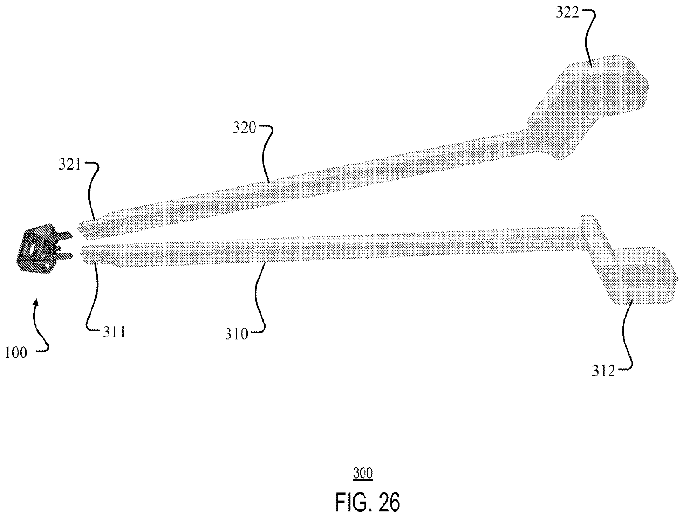

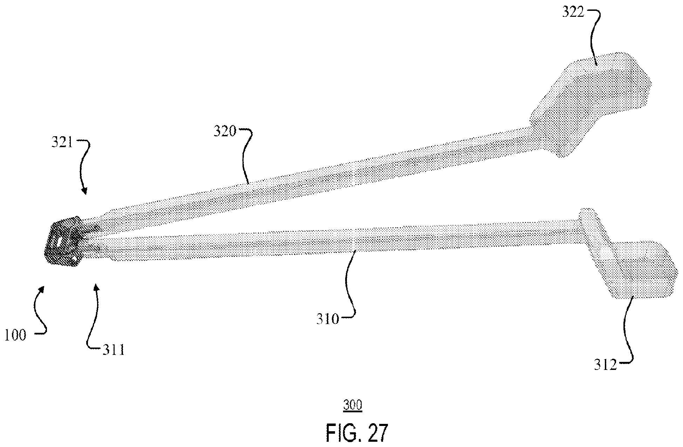

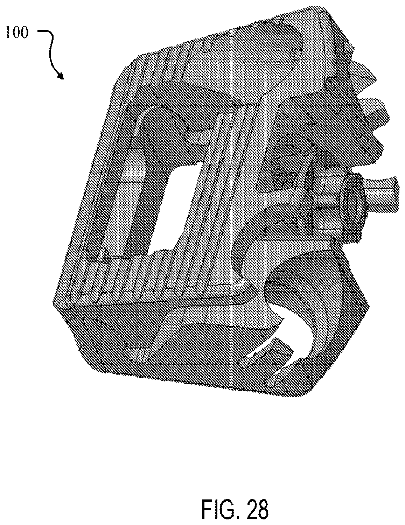

FIGS. 26 and 27 are various views of a surgical instrument 300 for use with disclosed expandable implants 100. In some embodiments, surgical instrument 300 may be referred to as a breakoff instrument and may be used to breakoff the mounting tangs 19, 29 of implant 100. In the example embodiment, surgical instrument 300 comprises a first instrument 310 and a second instrument 320. First instrument 310 may extend in a longitudinal direction from handle 312 towards gripping end 311. Similarly, second instrument 320 may extend in a longitudinal direction from handle 322 to gripping end 321. Gripping ends 311, 321 may comprise a channel having a size and shape generally corresponding to mounting tangs 19, 29. For example, as seen best in FIG. 27, the mounting tangs 19, 29 may be insert inside of the corresponding channels of gripping ends 311, 321. After the mounting tangs 19, 29 are nested within gripping ends 311, 321 an end user may push laterally outward and/or inward against handles 312, 322 to breakoff the corresponding mounting tang 19, 29. For example, as shown in FIG. 28 expandable implant 100 is in an expanded and lordosed configuration and the proximal portion of locking screw 50 and tangs 19, 29 have been broken off.

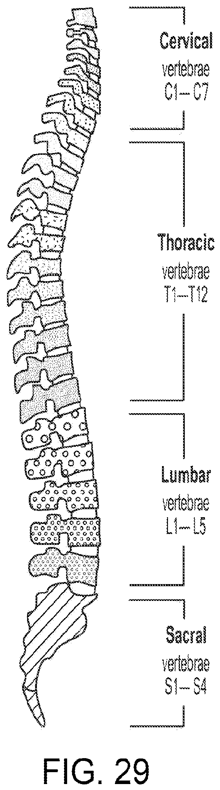

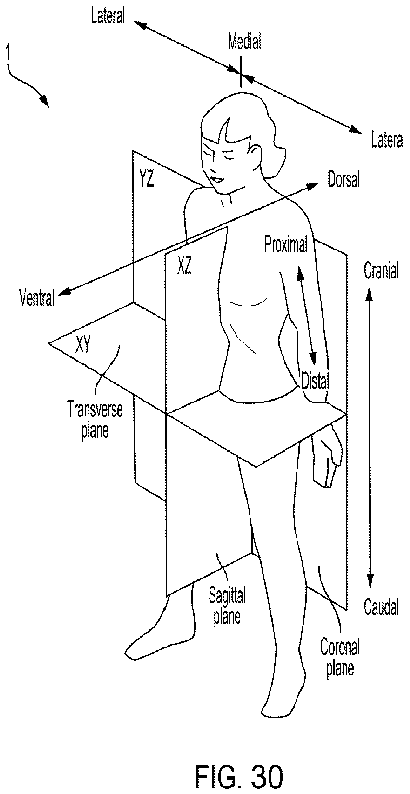

FIG. 29 is a reference drawing showing the human spine of which various disclosed implant embodiments may be installed in. FIG. 30 is a reference drawing showing various planes and reference directions of which the various disclosed implant embodiments may move in or act in with reference to a patient 1.

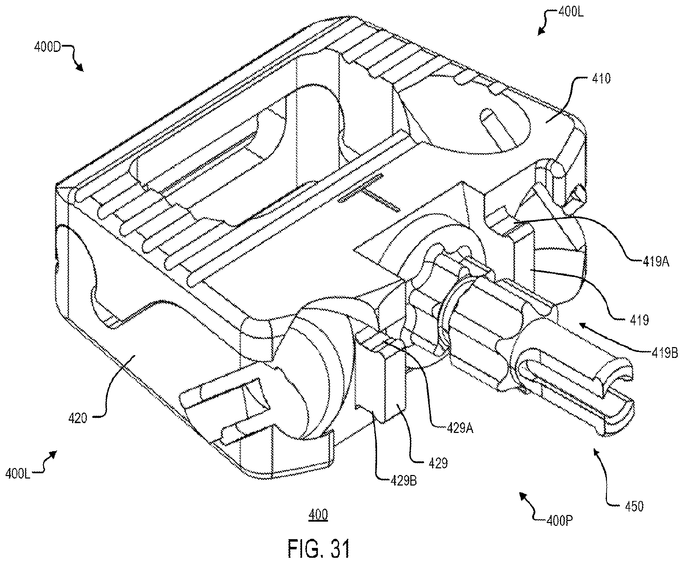

Referring generally to FIGS. 31-37 a second implant 400 embodiment is disclosed. Implant 400 may include the same, similar, and/or substantially the same components and functionality as explained above with respect to implant 100. Accordingly, duplicative description will be omitted. It shall be understood that various components and functionality of implant 100 are readily combinable with implant 400 and vice versa unless the context clearly indicates otherwise.

FIG. 31 is a perspective view of a second implant 400 embodiment. In this embodiment, implant 400 extends in a proximal-to-distal direction between a proximal end 400P and a distal end 400D and extends in a width-wise direction between a first lateral end 400L and a second lateral end 400L. Additionally, implant 400 includes a superior endplate 410 and an inferior endplate 420 having substantially similar features and functionality as explained above with respect to superior endplate 10 and inferior endplate 20 of implant 100, for example. However, in this embodiment, superior endplate 410 may include a first griping protrusion 419 extending in a proximal direction from the proximal end 400P of the superior endplate 410. Similarly, inferior endplate 420 may include a second griping protrusion 429 extending in a proximal direction from the proximal end 400P of the inferior endplate 420. In this embodiment, a size and shape of first gripping protrusion 419 is substantially the same as a size and shape of the second gripping protrusion 429. However, in other embodiments the first and second gripping protrusions 419, 429 may be differently sized and shaped, e.g., to bias the implant towards a surgical instrument and/or orientation for insertion. In this embodiment, and in the closed position, each gripping protrusion 419, 429 is disposed at approximately the same distance from an axis of rotation of breakoff set screw 450. In this embodiment, gripping protrusions 419, 429 may replace the need for the tangs 19, 29 of implant 100, for example. However, the concepts of utilizing breakoff tangs 19, 29 and gripping protrusions 419, 429 are not necessarily mutually exclusive and attributes of one may be combined and/or modified in view of the other.

In various embodiments, gripping protrusions 419, 429 may include various types of contouring to facilitate grasping of gripping protrusions 419, 429 with a corresponding inserter, for example surface indentations, surface outdents, channeling, etc. In the example embodiment, gripping protrusion 419 comprises a superior gripping surface 419A including an indented portion and an outdented chamfered portion at a proximal most end thereof, for example. Additionally, gripping protrusion 419 comprises an inferior gripping surface 419B including an indented portion and an outdented chamfered portion at a proximal most end thereof, for example. Likewise, gripping protrusion 429 comprises a superior gripping surface 429A including an indented portion and an outdented chamfered portion at a proximal most end thereof, for example. Additionally, gripping protrusion 429 comprises an inferior gripping surface 429B including an indented portion and an outdented chamfered portion at a proximal most end thereof, for example. In this way, gripping protrusions 419 and 429 are shaped like dovetails and a corresponding inserter tool may comprise a corresponding shaped dovetail groove which may grasp onto and/or slip over gripping protrusions 419 and 429 (not illustrated).

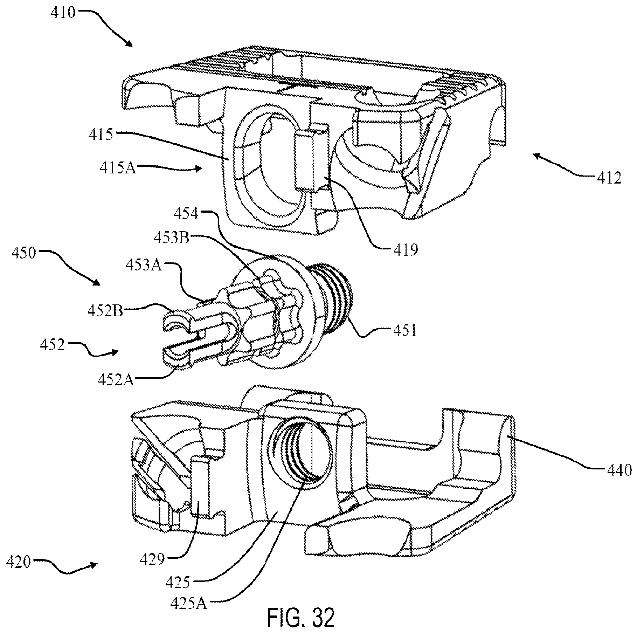

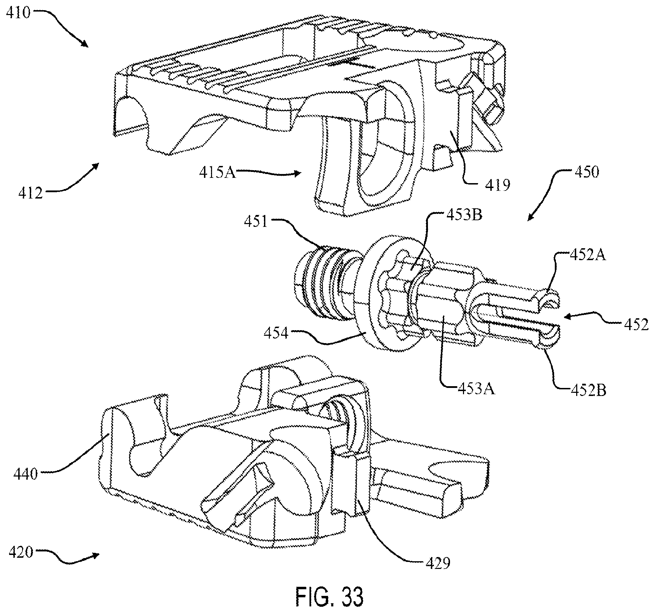

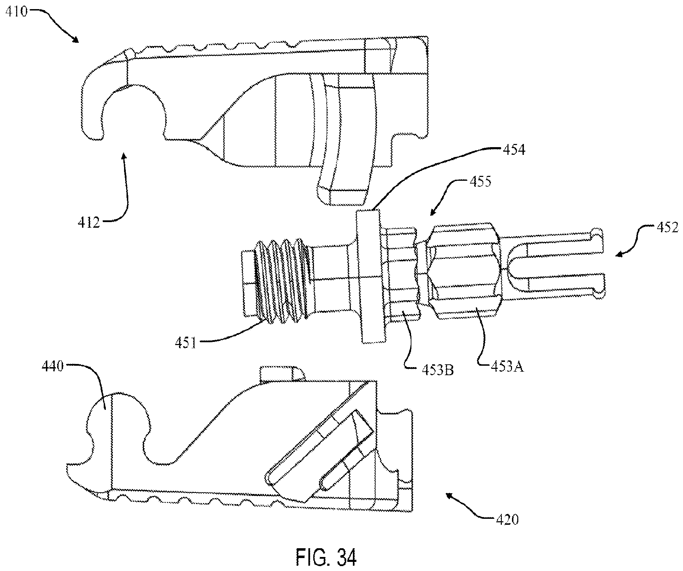

Referring generally to FIGS. 32, 33, and 34 various exploded parts views of implant 400 are illustrated. FIG. 32 is a first perspective exploded parts view of implant 400, FIG. 33 is a second perspective exploded parts view of implant 400, and FIG. 34 is a side exploded parts view of implant 400. In the example embodiment, the superior and inferior endplates 410, 420 of implant 400 may be hingedly coupled together by hinge member 440 and arcuate channel 412 having similar attributes as explained above with respect to hinge member 40 and channel 12 of implant 100, for example. Additionally, superior endplate 410 may also include a core 415 having an aperture 415A and inferior endplate 420 may include a core 425 having a threaded aperture 425A having similar attributes to core 15 and core 25 as explained above with respect to implant 100, for example. In the example embodiment, implant 400 utilizes a breakoff screw 450 for locking of a position of the superior and inferior endplate 410, 420. Breakoff screw 450 may include an external thread pattern 451 on an outside circumferential surface thereof, for example. The external thread pattern 451 of breakoff screw 450 may have a size and shape generally corresponding to the threaded aperture 425a of core 425 of the inferior endplate 420, for example. In various embodiments, an engagement surface 454 may be disposed adjacent and proximal of external thread pattern 451. In the example embodiment, engagement surface 454 is shaped like a washer and is directly connected to breakoff screw 450. However, in other embodiments, engagement surface 454 may be a washer or separated element, for example. In some embodiments, engagement surface 454 may be conically shaped. In the example embodiment, engagement surface 454 may include a relatively planar and/or flat distal surface and/or proximal surface.

In various embodiments, a proximal end of breakoff screw 450 may include a first flexible tang 452A and a second flexible tang 452B defining a discontinuous cylindrical shaped aperture 452 therebetween. Additionally, the first flexible tang 452A and second flexible tang 452B may each include an outdent at a proximal end thereof that is shaped like a segment of an annular ring. In the example embodiment, the first flexible tang 452A may flex inward towards the second flexible tang 452B under loading and vice versa due to the gap between them. At least one advantage of this configuration is that it may facilitate securing breakoff screw 450 to a corresponding drive tool (not illustrated) and the retention of the broken off part. For example, a drive tool may comprise a drive end having a female cavity with a corresponding size and shape to the drive features 453A, 453B. In various embodiments, the cavity may include a pair of indentations corresponding in size and shape to the outdents of flexible tangs 452A and 452B, for example. In use, an end user may align the flexible tangs 452A, 452B with the cavity, push down against the flexible tangs 452A, 452B which may cause them to flex inward towards each other such that they may slide within the cavity until the outdents of flexible tangs 45A and 452B are seated within the corresponding indents of the drive tool. Thereafter, an end user may continue to rotate and or tighten breakoff screw 450. In this way, after a proximal portion of breakoff screw 450 is broken off it may remain retained by the inserter due to the flexible tangs 452A and 452B being seated within the corresponding indents.

In some embodiments, aperture 452 may be understood as a cylindrical protrusion extending in a proximal direction and having a first slit and a second slit extending along the length thereof such that the cyldrical protrusion is compressible. In the example embodiment, a first drive feature 453A and a second drive feature 453B may be disposed adjacent to and distally with respect to a proximal most end of breakoff screw 450. Additionally, first and second drive features 453A, 453B may be disposed proximally with respect to engagement surface 454. In various embodiments, a breakoff location may be positioned between and/or adjacent to drive features 453A, 453B, which will be explained in further detail below. In the example embodiment, drive features 453A, 453B take a hexalobular shape, although various other shapes such as hexaganol, polygonal, torx, etc. are also contemplated. In some embodiments, a surgical drive tool having a corresponding socket may be coupled to drive features 453A and/or 453B to cause rotation of breakoff screw 450. Similarly as explained above with respect to locking screw 50, once breakoff screw 450 has been sufficiently tightened a proximal portion may breakoff and/or shear off while the distal portion may remain coupled to implant 400 locking a relative orientation of superior endplate 410 and inferior endplate 420 in place.

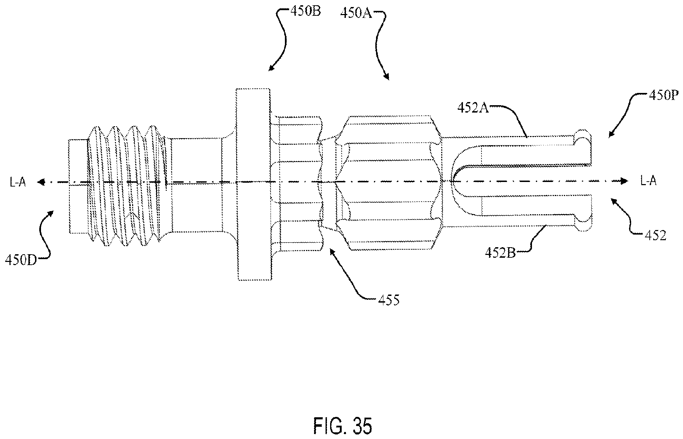

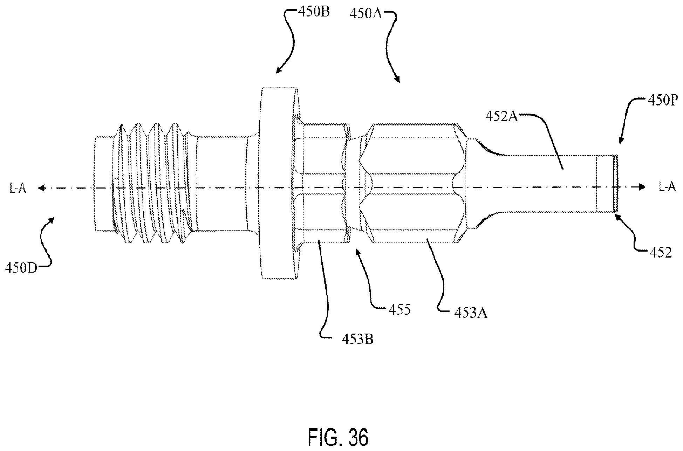

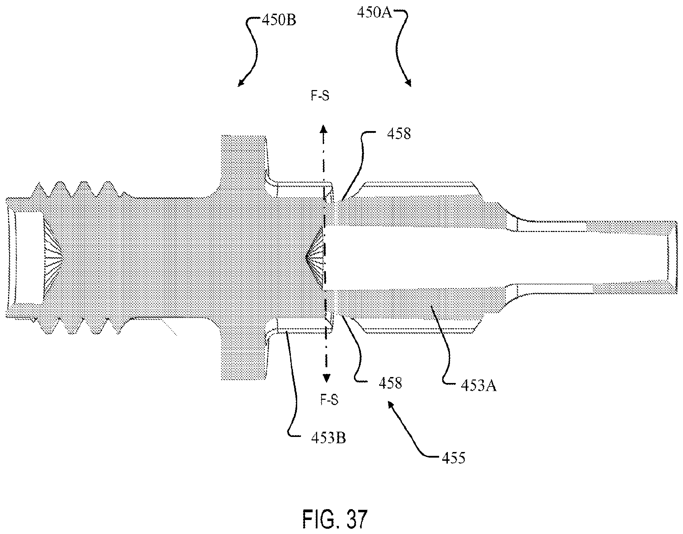

As seen best in FIGS. 35-37, breakoff screw 450 may extend in a longitudinal direction along a longitudinal axis L-A that is coaxially aligned with breakoff screw 450. FIG. 35 is a first side view of breakoff screw 450 in which the superior surface 452A and inferior surface 452B of discontinuous aperture 452 are visible. FIG. 36 is a second side view of a breakoff screw 450 that is rotated about 90 degrees with respect to FIG. 35 in which only the superior surface 452A is visible. With reference to FIG. 37, in various embodiments, breakoff location 455 may comprise a recessed fracture surface F-S that is inset with respect to a leading edge (proximal most edge) of drive feature 453A. At least one advantage of the recessed fracture surface may be that delicate tissue is prevented and/or suppressed from coming into contact with relatively sharp ends of the fracture surface. In the example illustration, a relative location of the recessed fracture surface is represented by dashed lines F-S. In the example embodiment, breakoff location 455 may be considered as the boundary between a proximal portion 450A and a distal portion 450B of breakoff screw 450, for example. In this embodiment, the boundary between drive features 453A and 453B comprises a necked down portion 458 extending from a distal end of drive feature 453A to an inset portion of drive feature 453B that is inset with respect to an outermost and/or proximal most surface of drive feature 453B thereby defining a portion of breakoff set screw 450 having a minimum cross sectional diameter. Accordingly, when breakoff screw 450 is sufficiently tightened within threaded aperture 425A of core 425 such that the breakoff location 455 experiences a sufficient torque the proximal portion 450A may breakoff from the distal portion 450B. For example, when a sufficient rotational force is applied to the proximal end of breakoff screw 450 while a distal end of breakoff screw 450 is stationary, i.e., when breakoff screw 450 is secured in a locked position and a continued rotational force (torque) is applied to the proximal end of breakoff screw 450 the drive feature 453A and cylindrical end having the discontinuous aperture 452 may breakoff. For further explanation in the similar context of implant 100, see FIGS. 10 and 11 and the corresponding discussion thereof.

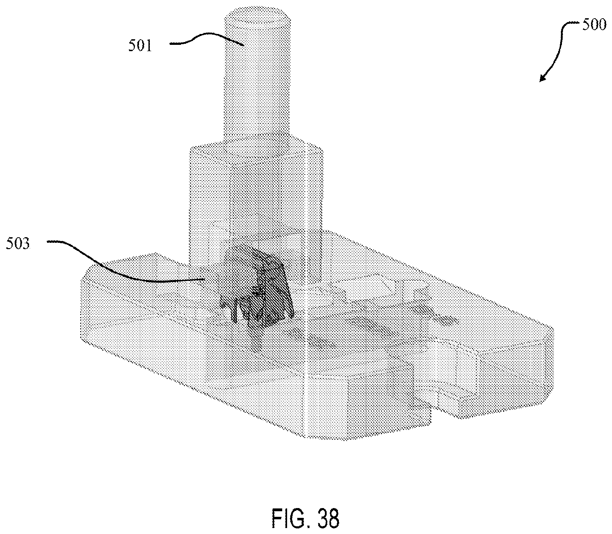

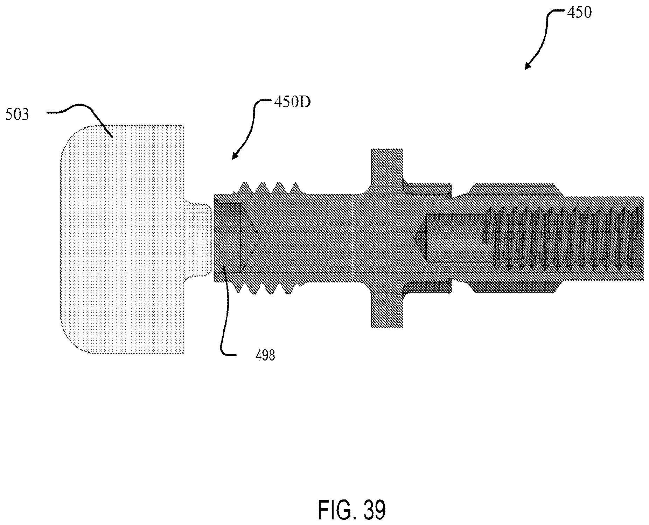

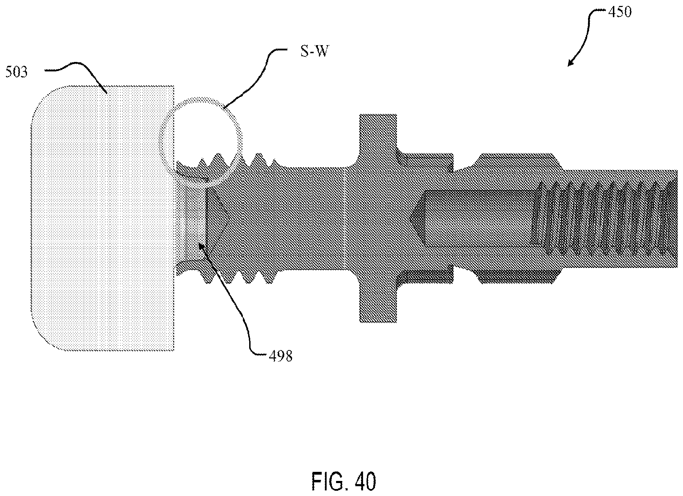

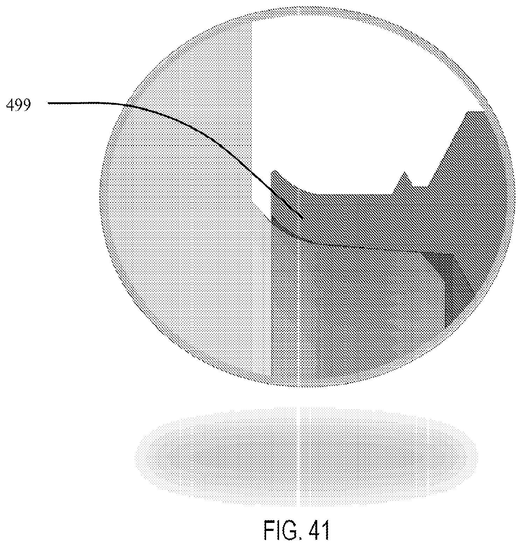

Referring to FIGS. 38-42 an example swaging process is performed to a distal most end of breakoff screw 450. FIG. 38 is a perspective view of a swaging fixture 500, and FIG. 39 is a cross section view of a swage mandrel and a distal end 450D of a breakoff screw 450 before the commencement of a swage process. FIG. 40 is a cross section view showing a result of a swage process, and FIG. 41 is an enlarged view of region S-W of FIG. 40. In the example embodiment, swaging fixture 500 comprises a swaging ram 501 and a swaging mandrel 503 that are supported by the base of the apparatus. The swaging mandrel 503 may include an outdent that corresponds to and is slightly larger than the distal most indent 498 (swage bore) of breakoff screw 450, for example. As seen in region S-W of FIG. 40, when swaging mandrel 503 is advanced into the distal most indent 498 (swage bore) a flared out portion 499 (swaged portion) is formed at a distal most end of breakoff screw 450. An example advantage of a swaged end may be that it facilitates retention of the breakoff screw 450 such that it serves as a stopping structure preventing breakoff screw 450 from backing out of implant 100.

It shall be understood that although breakoff screw 450 is explained concurrently with implant 400 and in the context of an intervertebral implant, the concepts of breakoff screw 450 may be applied to other embodiments used for alternate purposes, for example for use in a pedicle screw to insert in a tulip to tighten down a rod. It should be understood that various aspects disclosed herein may be combined in different combinations than the combinations specifically presented in the description and accompanying drawings. For example, features, functionality, and components from one embodiment may be combined with another embodiment and vice versa unless the context clearly indicates otherwise. Similarly, features, functionality, and components may be omitted unless the context clearly indicates otherwise. It should also be understood that, depending on the example, certain acts or events of any of the processes or methods (not claimed) described herein may be performed in a different sequence, may be added, merged, or left out altogether (e.g., all described acts or events may not be necessary to carry out the techniques).

Referring generally to FIGS. 42-51, a third implant 400Z and a surgical tool 600 for use with various implants is disclosed. FIGS. 42A-42B illustrate a third embodiment of an implant 400Z. Implant 400Z may have the same, similar, and/or substantially the same components and functionality as explained above with respect to implant 400, for example. Accordingly, duplicative description will be omitted. A difference may be that breakoff screw 450 does not include the discontinuous cylindrical shaped aperture 452 (see FIG. 34) having tangs 452A, 452B, for example. Rather, breakoff screw 450, to the extent included, may include a continuous cylindrical shaped aperture 452Z without the discontinuity. In some embodiments, a similar screw may be used that is not a breakoff screw but rather may be referred to as a locking screw. Additionally, in this embodiment, implant 400Z may include a first gripping indentation 419Z and a second gripping indentation 429Z (see FIG. 42A) in lieu of the gripping protrusions 419, 429 (as shown in FIG. 33), for example. In various embodiments, first gripping indentation 419Z may be formed as a part of the superior endplate 410 and second gripping indentation 429Z may be formed as a part of the inferior endplate 420, for example.

In various embodiments, gripping indentations 419Z, 429Z may include various types of contouring to facilitate coupling with a corresponding surgical tool 600. In this embodiment, gripping indentations 419Z, 429Z each comprise a slotted indentation extending in a proximal to distal direction with a curved superior surface 419S, 429S and a curved inferior surface 419I, 429I. Additionally, gripping indentations 419Z, 429Z each have an open channel portion 419X, 429X adjacent the breakoff screw 450, to the extent a breakoff screw is included. As will be explained in further detail below, corresponding gripping protrusions of a surgical tool 600 may have a substantially similar size and shape to the gripping indentations 419Z, 429Z, for example.

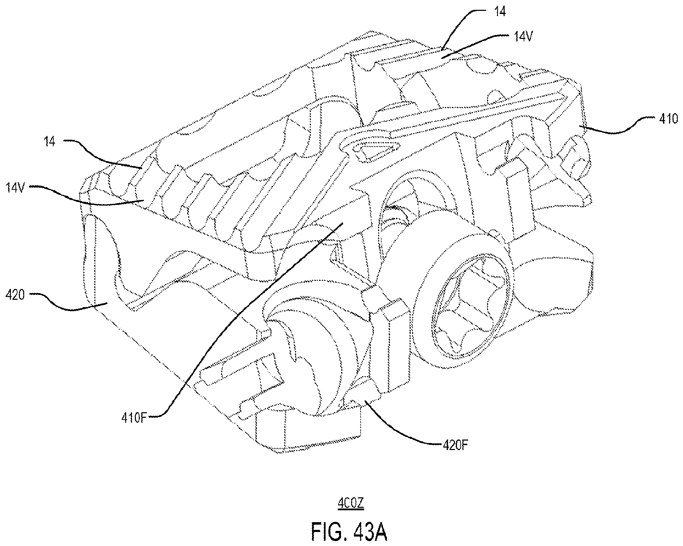

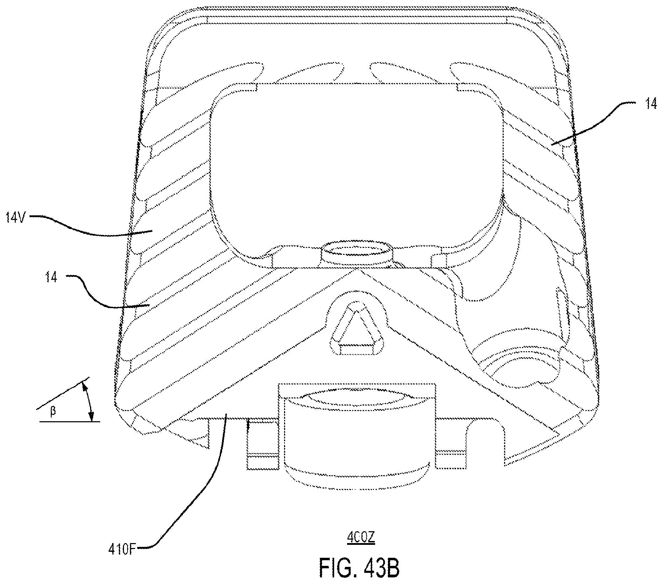

FIGS. 43A-43B illustrate a similar embodiment as FIGS. 42A-42B. As shown, the implant 400Z includes angled engagement features 14. In the example embodiment, engagement features 14 extend diagonally across the exposed uppermost surface of superior endplate 410 and across the exposed lowermost surface of inferior endplate 420. The engagement features 14 comprise flattened top rails that are sequentially spaced apart with rounded bottom valleys 14V therebetween. As seen best in the top down view of FIG. 43B, the engagement features 14 of the superior endplate 410 are oriented at an angle β with respect a proximal face 410F of the superior endplate 410. Similarly, the engagement features 14 of the inferior endplate 420 are oriented at an angle β with respect a proximal face 420F of the inferior endplate 420 (see FIG. 43A). In various embodiments, the angle β may be about 20-40 degrees and in at least one embodiment the angle β may be angled at about 30 degrees. At least one advantage of this orientation may be a relatively greater resistance and/or suppression of expulsion of the implant 400Z relative to embodiments in which engagement features solely extend horizontally across the implant 400Z. In the example embodiment of FIGS. 43A-43B, by orienting the engagement features 14 diagonolly, the implant 400Z may resist expulsion in multiple directions, e.g., forward flexion/extension and lateral bending.

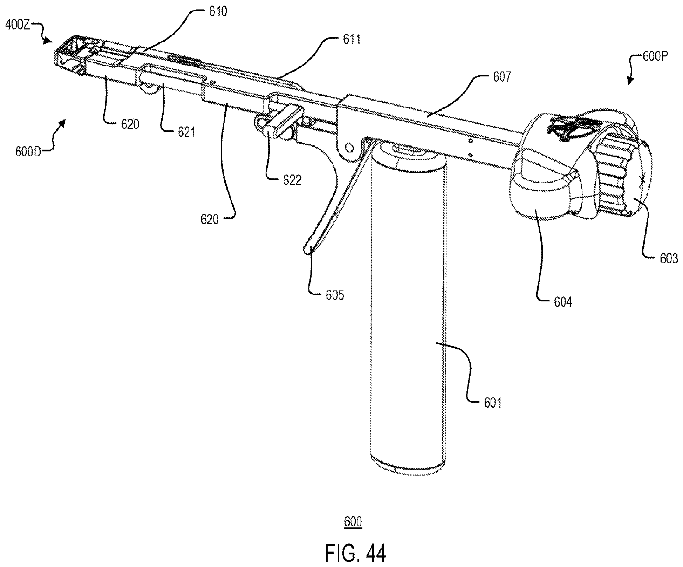

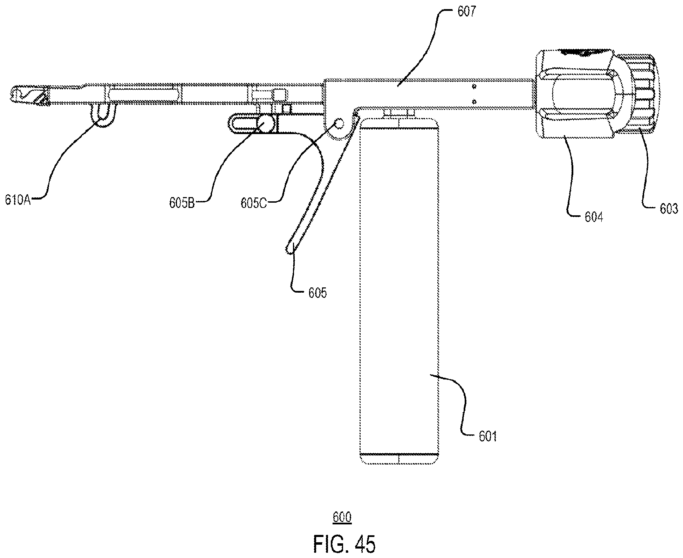

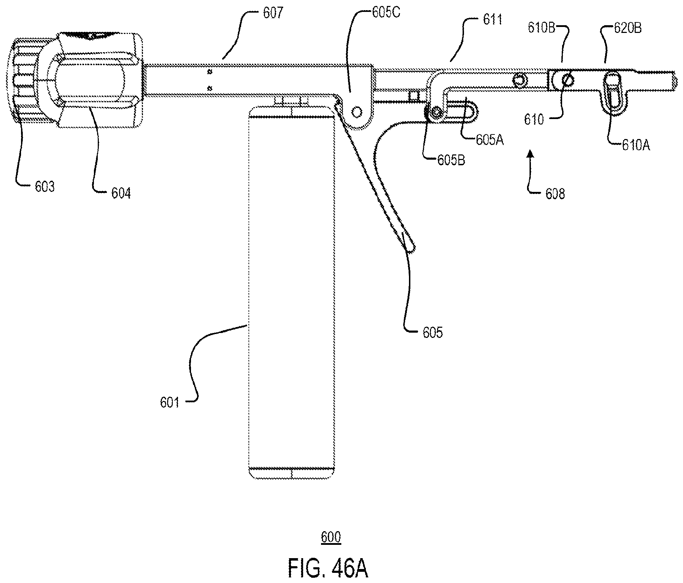

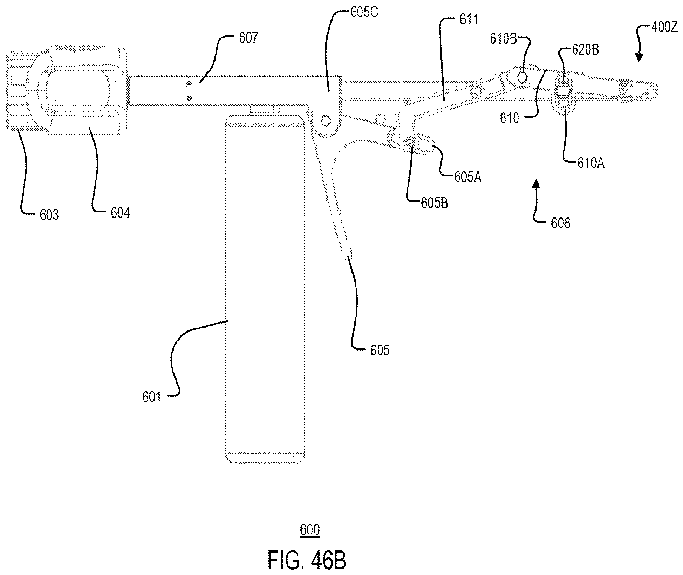

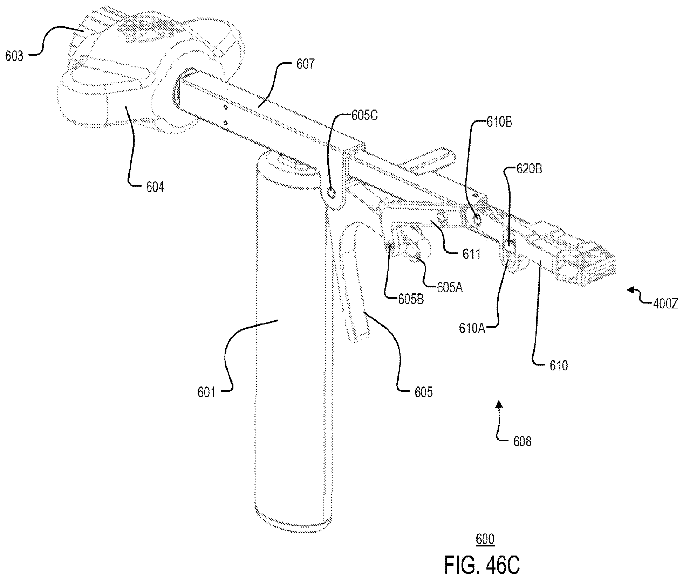

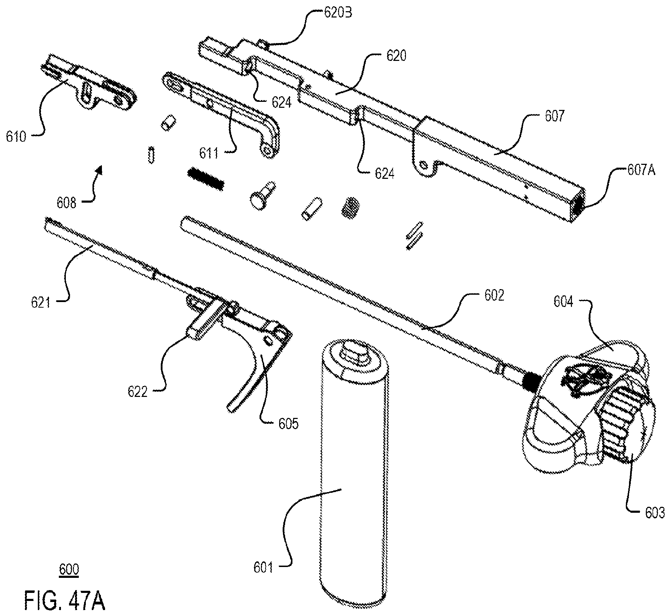

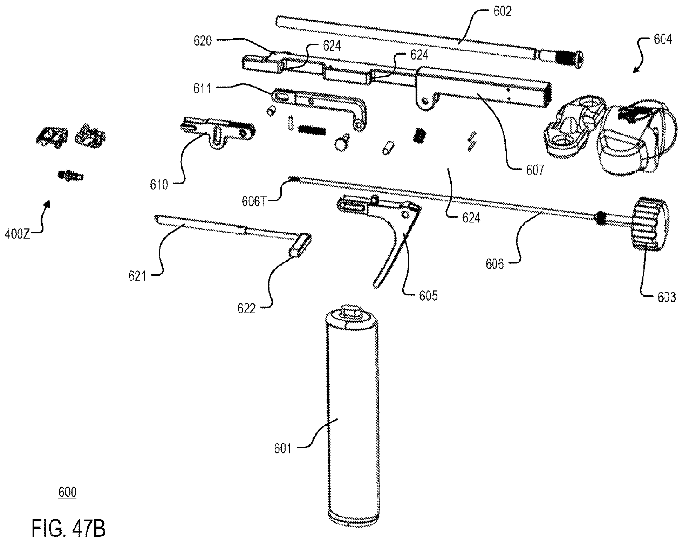

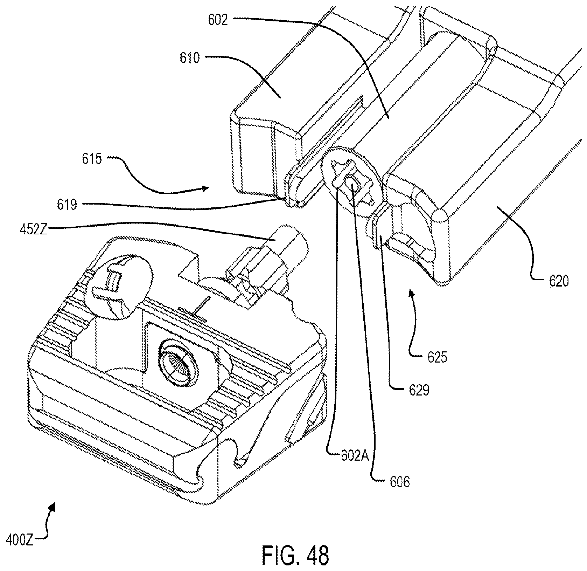

Referring generally to FIGS. 44-48, various views of a surgical tool 600 are disclosed. FIG. 44 illustrates the surgical tool 600 coupled to implant 400Z. FIG. 44 is a perspective view of surgical tool 600; FIG. 45 is a first side view of surgical tool 600; and FIG. 46A is a second side view of surgical tool 600. FIG. 46B is a third side view of surgical tool 600 in an operative position and FIG. 46C is a perspective view of surgical tool 600 in the operative position of FIG. 46B. FIG. 47A is an exploded parts view of surgical tool 600 with select parts removed for ease of understanding and FIG. 47B is an exploded parts view of surgical tool 600 showing additional detail and parts. FIG. 48 illustrates surgical tool 600 immediately prior to coupling with implant 400Z. In the example embodiment, tool 600 may serve several purposes. For example, tool 600 may be used to insert implant 400Z, expand implant 400Z, and in some embodiments may also rotate breakoff screw 450 to cause it to separate into two portions along breakoff location 455 (see FIG. 34).

With reference to the perspective view of FIG. 44 and the exploded parts views of FIGS. 47A and 47B, tool 600 may include a gripping handle 601 that is securely coupled to first body portion 607. First body portion 607 may rotatably support an inner shaft 606 and an outer shaft 602 therein. Shafts 606, 602 may extend through aperture 607A of first body portion 607 in a longitudinal direction from a proximal end to a distal end. The inner shaft 606 may be securely connected to turn knob 603 at a proximal end 600P and the outer shaft 602 may be securely connected to turn handle 604 at the proximal end 600P. The inner shaft 606 may extend through outer shaft 602 and include a thread pattern 606T at a distal end thereof for securely connecting to corresponding threads 456 of breakoff screw 450 (see FIG. 42B for example). The inner shaft 606 may be independently rotatable relative to outer shaft 602 by rotating turn knob 603. Additionally, the outer shaft 602 may be independently rotatable relative to inner shaft 606 by rotating turn handle 604, for example. Generally, both the inner shaft 606 and outer shaft 602 will interact with and/or couple to breakoff screw 450 as will be explained in further detail below.

With reference to FIGS. 45 and 46A-46C, tool 600 may include an actuator 605 in the form of a trigger that is connected to first body portion 607 at pin 605C. Actuator 605 may be actionable to expand implant 400Z by pivoting a linkage assembly 608 including first arm 610 and second arm 611 relative to a third fixed arm 620, for example. Second arm 611 may be coupled to actuator 605 via pin 605B such that pin 605B may be linearly translatable forward and backward in a proximal-to-distal direction within slotted aperture 605A. In turn, second arm 611 may be coupled to first arm 610 via pin 610B such that second arm 611 may pivot up and down. Additionally, first arm 610 may be coupled to third arm 620 and in various embodiments third arm 620 may be fixed relative to first body portion 607. Additionally, third arm 620 may include a protrusion 620B which may be slidably seated within a curved vertical slot, for example slot 610A, and thereby guide motion of arm 610 as it pivots up and down. As seen best in FIGS. 46B and 46C, depressing actuator 605 may cause linkage assembly 608 to pivot by causing second arm 611 to move distally such that first arm 610 pivots relative to third arm 620 and implant 400Z may be expanded.

With reference back to FIG. 44, third arm 620 may include an aperture extending therethrough in a proximal to distal direction for accommodating a linearly translatable shaft 621. Shaft 621 may independently move forward and backward in a proximal to distal direction within the through aperture 624 of third fixed arm 620 (see FIGS. 47A and 47B), for example. Shaft 621 may include a charging handle at a proximal end thereof allowing an end user to manipulate shaft 621 in a forward and backward motion (proximal motion and distal motion in the proximal-to-distal direction). In some embodiments, an end user may translate charging handle 622 rotatably in a clockwise or counterclockwise motion, for example to lock shaft 621 in a relative position. Additionally, in some embodiments, when shaft 621 is not in a locked position, depressing actuator 605 will not expand implant 400Z for reasons explained in more detail below.

With reference to FIG. 48, first arm 610 may include a gripping outdent 619 having a size and shape generally corresponding to a size and shape of gripping indentation 419Z. Additionally, first arm 610 may include a counter torque surface 615 having a size and shape generally corresponding to a size and shape of the proximal surface of the superior endplate 410, for example. Similarly, shaft 621, extending through fixed arm 620, may include a gripping outdent 629 having a size and shape generally corresponding to a size and shape of gripping indentation 429Z (as shown in FIG. 42A). Additionally, second arm 620 may include a counter torque surface 625 having a size and shape generally corresponding to a size and shape of the proximal surface of the inferior endplate 420, for example. In this way, when shaft 621 is moved distally such that the outdent 629 is seated within indent 429Z and outdent 619 is seated within indentation 419Z (as shown in FIG. 42A) an end user may activate actuator 605 to expand implant 400Z while surfaces 615, 625 resist a twisting motion of implant 100. For example, as shown in FIGS. 46B and 46C an end user may depress actuator 605 by squeezing the trigger which in turn may cause the linkage assembly 608 to pivot the first arm 610 relative to fixed arm 620 such that tool 600 may urge the superior endplate 410 and inferior endplate 420 away from one another.

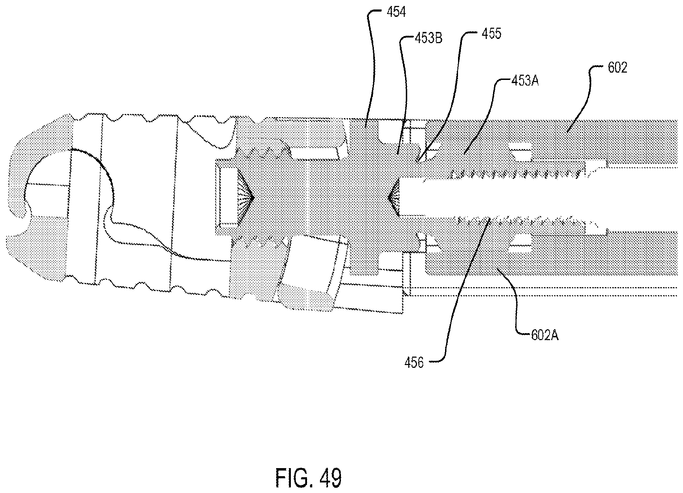

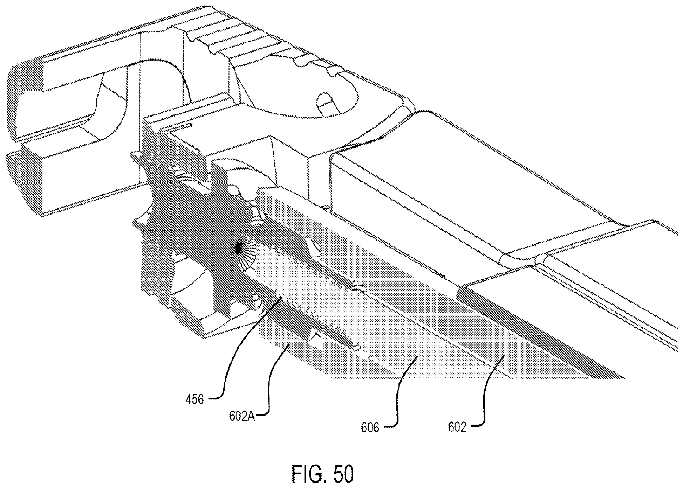

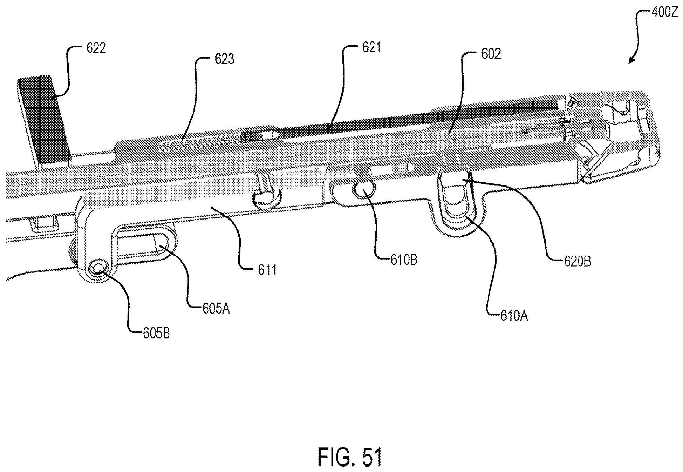

With reference to the cross-section drawings of FIGS. 49, 50, and 51 additional features and functionality of tool 600 will be explained. In FIGS. 49 and 50, it is shown that the threaded end of inner shaft 606 is threadably engaged with the threaded aperture 456 of breakoff screw 450. Additionally, a proximal drive feature 453A is mated within a corresponding female drive feature 602A (see FIG. 48) at the distal most end of outer shaft 602. As explained previously, inner shaft 606 and outer shaft 602 are independently operable. In use, an end user may initially position inner shaft 606 adjacent to / within the front portion of threaded aperture 452 and rotate inner shaft 606 via turn knob 603. As inner shaft 606 is rotated, implant 400Z is pulled towards tool 600. FIGS. 49-51 show implant 400 after inner shaft 606 has been sufficiently rotated such that implant 400Z abuts the counter torque surfaces 615, 625 and the gripping protrusion 619 may be seated within the corresponding gripping indentation 419Z. With reference to FIG. 51, once implant 400Z is pulled sufficiently towards tool 600, an end user may actuate shaft 621 by pushing on charging handle 622 to overcome the biasing force of spring 623 such that gripping protrusion 629 may be seated within the corresponding gripping indentation 429Z, for example. In some embodiments, an end user may rotate charging handle 622 such that biasing force of spring 623 is prevented from urging shaft 621 away from implant 400Z. At this point, the implant 400Z may be in an operatively engaged position with tool 600.

Once implant 400Z is in the operatively engaged position, an end user may insert implant 400Z into a disc space between a superior vertebra and inferior vertebra (cephalad vertebra and caudal vertebra), for example. After the implant 400Z is positioned between the superior and inferior vertebrae, an end user may depress actuator 605 thereby causing the linkage assembly 608 to pivot and spread the superior endplate 410 apart from the inferior endplate 420. Once the end user has expanded implant 400Z to an appropriate position, the end user may rotate turn handle 604 which will rotate breakoff screw 450 via first drive feature 453A. The end user may continue to rotate breakoff screw 450 such that it advances into implant 400Z in a proximal to distal direction and locks implant 400Z in the expanded configuration similarly as explained above. The end user may continue to rotate turn handle 604 until the torque applied to breakoff screw 450 is great enough that the breakoff screw 450 will shear into two pieces similarly as explained above. Notably, the broken off or sheared off portion of breakoff screw 450 may be retained by tool 600 on account of inner shaft 606 being threadably coupled to threaded aperture 452.

Unless otherwise specifically defined herein, all terms are to be given their broadest possible interpretation including meanings implied from the specification as well as meanings understood by those skilled in the art and/or as defined in dictionaries, treatises, etc. It must also be noted that, as used in the specification and the appended claims, the singular forms "a," "an" and "the" include plural referents unless otherwise specified, and that the terms "comprises" and/ or "comprising," when used in this specification, specify the presence of stated features, elements, and/or components, but do not preclude the presence or addition of one or more other features, steps, operations, elements, components, and/or groups thereof.

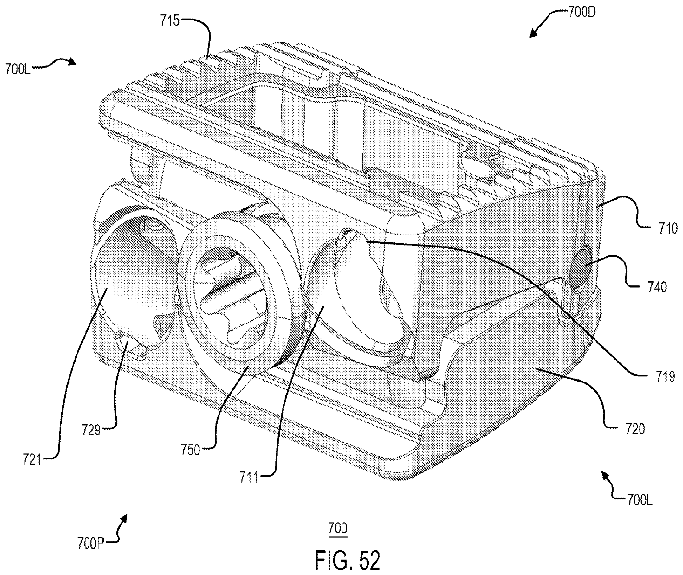

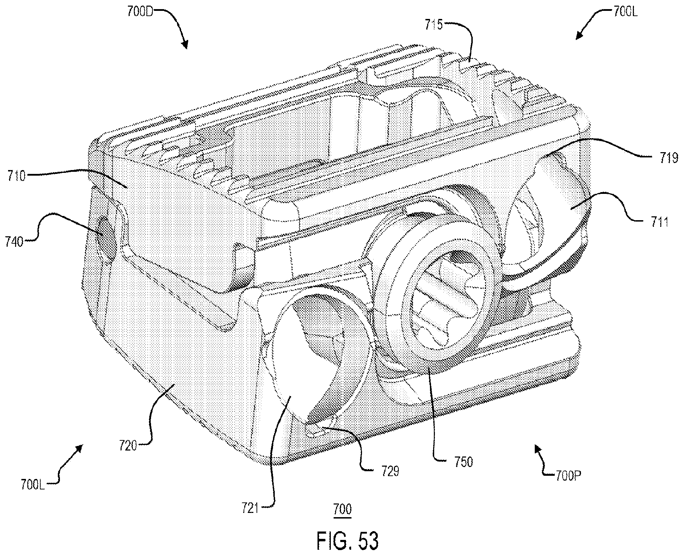

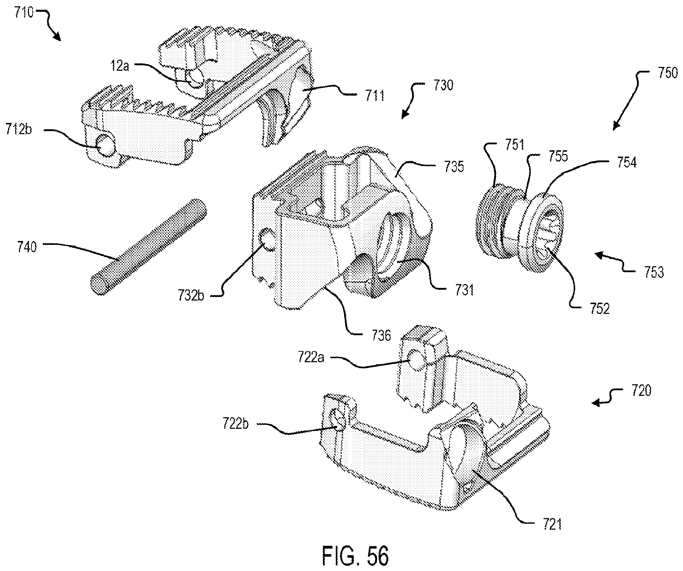

Referring to FIGS. 52-62 generally, various spinal implants 700 are disclosed. The components of spinal implant 700 can be fabricated from biologically acceptable materials suitable for medical applications, including metals, synthetic polymers, ceramics and bone material and/or their composites. For example, the components of spinal implant 700, individually or collectively, can be fabricated from materials such as stainless steel alloys, commercially pure titanium, titanium alloys, Grade 5 titanium, super-elastic titanium alloys, cobalt-chrome alloys, superelastic metallic alloys (e.g., Nitinol, super elasto-plastic metals, such as GUM METAL^{®}), ceramics and composites thereof such as calcium phosphate (e.g., SKELITE^{™}), thermoplastics such as polyaryletherketone (PAEK) including polyetheretherketone (PEEK), polyetherketoneketone (PEKK) and polyetherketone (PEK), carbon-PEEK composites, PEEK-BaSO4 polymeric rubbers, polyethylene terephthalate (PET), fabric, silicone, polyurethane, silicone-polyurethane copolymers, polymeric rubbers, polyolefin rubbers, hydrogels, semi-rigid and rigid materials, elastomers, rubbers, thermoplastic elastomers, thermoset elastomers, elastomeric composites, rigid polymers including polyphenylene, polyamide, polyimide, polyetherimide, polyethylene, epoxy, bone material including autograft, allograft, xenograft or transgenic cortical and/or corticocancellous bone, and tissue growth or differentiation factors, partially resorbable materials, such as, for example, composites of metals and calcium-based ceramics, composites of PEEK and calcium based ceramics, composites of PEEK with resorbable polymers, totally resorbable materials, such as, for example, calcium based ceramics such as calcium phosphate, tri-calcium phosphate (TCP), hydroxyapatite (HA)-TCP, calcium sulfate, or other resorbable polymers such as polyaetide, polyglycolide, polytyrosine carbonate, polycaroplaetohe and their combinations.

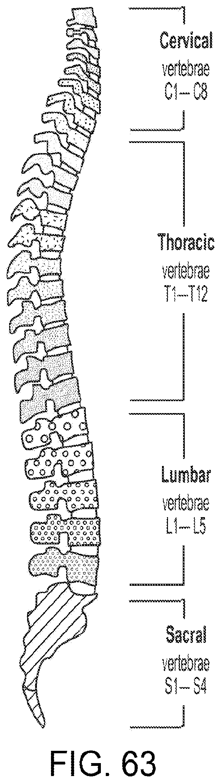

Referring generally to FIGS. 52-62 various embodiments of an expandable implant 700 are disclosed. FIGS. 63-64 are reference drawings showing the human spine and various medical terminology as it relates to planes and directions of which various components of implant 700 may act or move in.

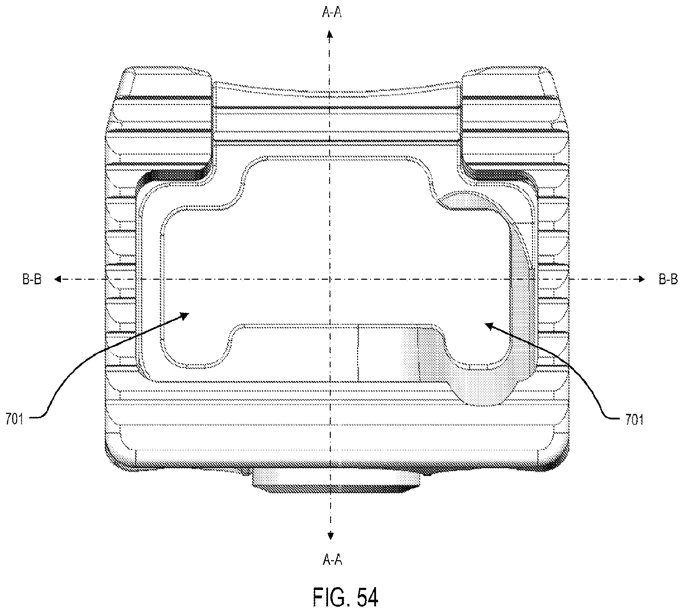

FIGS. 52-53 illustrate example perspective views of an expandable implant 700 in a partially expanded position and FIG. 54 is a top down view of an expandable implant 700. As illustrated, expandable implant 700 may include a proximal end 700p, a distal end 700d, and first and second lateral sides 700l. The proximal end 700p may include a screw guide aperture 731 and a pair of gripping indentations 719, 729 on opposite sides of the screw guide aperture 731, for example. Additionally, a pair of bone screw apertures 711, 721 may be positioned on the proximal end 700p, for example. In various embodiments, and as illustrated in FIGS. 52-53, the gripping indentations 719, 729 may be formed as a cutout portion adjoining the bone screw apertures 711, 721, for example. Implant 700 may be referred to as an externally expandable implant because an end user such as a surgeon may use a surgical tool to open and close implant 700, for example an external tool may adjust the lordotic angle of implant 700. Once implant 700 is expanded to an appropriate lordotic angle (also referred to as angle of inclination), an end user may fix the relative angle of the superior endplate 710 relative to the inferior endplate 720 by tightening locking screw 750, for example. At least one advantage of relying on an external tool to adjust a lordotic angle of implant 700 may be the reduction of internal components within implant 700 relative to other forms of implants relying on various moving mechanisms and/or expansion mechanisms, for example. Accordingly, in various embodiments, implant 700 may have a relatively large void space in the interior thereof, which may facilitate a fusion process during an ACDF procedure. For example, implant 700 may have a relatively large internal space for packing of bone growth promoting materials and/or bone grafts.

As illustrated in FIG. 54, implant 700 may extend in a proximal-to-distal direction from the proximal end 700p to the distal end 700d though axis A-A through the center of the implant 700, for example. Implant 700 may extend in a widthwise direction (also referred to as lateral direction) from the first lateral side 700l to the second lateral side 700l through axis B-B through the center of the implant 700, for example. The axis A-A may be perpendicular and/or substantially perpendicular to the axis B-B. For example, the proximal-to-distal direction may be perpendicular to the widthwise direction.

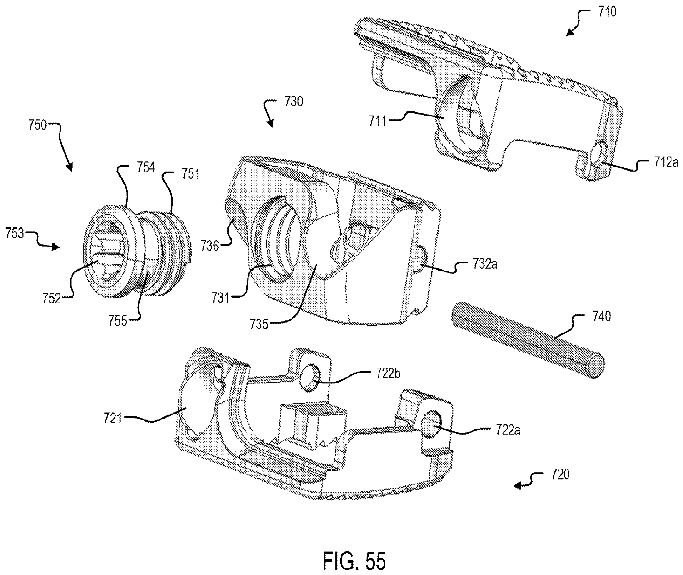

FIGS. 55-56 illustrate example exploded parts views of an expandable implant 700. Implant 700 may include a superior endplate 710 and an inferior endplate 720 defining the top and bottom surfaces of implant 700, for example. The superior endplate and inferior endplate 710, 720 may be hingedly coupled to one another via pin 740, for example. The superior and inferior endplates 710, 720 may be adjustable with respect to one another in the vertical direction and inclinable with respect to one another, i.e., capable of distraction and lordosis by rotation around pin 740, for example. Additionally, a core 730 may be disposed centrally within implant 700 and the superior endplate 710 and inferior endplate 720 may be hingedly coupled to the core 730 via pin 740, for example. Pin 740 may extend in the lateral direction through pin receiving apertures 712a, 712b of superior endplate 710, pin receiving apertures 732a, 732b of core 730, and pin receiving apertures 722a, 722b of inferior endplate 720, for example. In some embodiments, pin 740 may be referred to as a rod or a dowel, for example. Additionally, in some embodiments, the superior endplate 710, inferior endplate 720, and core 730 may collectively be referred together as an expandable body.

In some embodiments, pin 740 may be "press fit" to core 730 by extending through pin receiving apertures 732a, 732b (may also be referred to as an interference fit). As used herein, the terms "press fit" and "interference fit" are intended to have their ordinary technical meaning, for example a form of fastening between two tight fitting mating parts that produces a joint which is held together by friction after the parts are pushed together. In some embodiments, the connection of pin 740 to core 730 may be a press fit or interference fit where the components are tightly held together such that the core 730 may not rotate relative to pin 740 and/or pin 740 may be fixed in position relative to core 730, for example. At least one advantage of utilizing a press fit connection may be that the connection assures rigid, permanent support for pin 740 at each tension point defined by pin receiving apertures 732a, 732b with no relative movement thereby reducing wear and/or fatigue while providing a shaft and/or pivot point for superior endplate 710 and inferior endplate 720 to rotate about. However, in other embodiments, some rotation may be possible.

In various embodiments, pin 740 may be "slip fit" to superior endplate 710 by extending through pin receiving apertures 712a, 712b, for example. Similarly, in various embodiments pin 740 may be "slip fit" to inferior endplate 720 by extending through pin receiving apertures 722a, 722b, for example. As used herein, the term "slip fit" is intended to have an ordinary technical meaning, for example, a form of fastening between two relatively loose but snug mating parts that produces a joint which allows rotation and/or movement.

The proximal end 700p of superior endplate 710 may include a first bone screw aperture 711 extending through the upper surface of superior endplate 710 for engaging with a superior vertebra, for example. In the example embodiment, the first bone screw aperture 711 extends from the proximal end 700p of superior endplate 710 through a bone graft aperture 701 of superior endplate 710 (see FIG. 54). Additionally, core 730 may include a first bone screw cutout 735 comprising an arcuate channel for accommodating a bone screw extending through the first bone screw aperture 711, for example.

Similarly, the proximal side 700p of inferior endplate 720 may include a second bone screw aperture 721 extending through the lower surface of inferior endplate 720 for engaging with an inferior vertebra, for example. In the example embodiment, the second bone screw aperture 721 extends from the proximal end 700p of inferior endplate 720 through a bone graft aperture 701 of inferior endplate 720 (see FIG. 54). Additionally, in various embodiments, each of superior endplate 710 and inferior endplate 720 may include a bone graft aperture 701 having substantially the same size and shape, for example. Additionally, core 730 may include a second bone screw cutout 736 comprising an arcuate channel for accommodating a bone screw extending through the second bone screw aperture 721, for example.

In various embodiments, core 730 may include a screw guide aperture 731 (also referred to as a locking screw guide aperture). Screw guide aperture 731 may be disposed in a central position of implant 700 at proximal end 700p, for example. Screw guide aperture 731 may include a female thread pattern having a size and shape corresponding to a male thread pattern 751 of locking screw 750, for example. Screw guide aperture 731 may rotatably support a locking screw 750 therein such that rotation of locking screw 750 may cause linear translation of locking screw 750 in the proximal-to-distal direction along axis A-A, for example.

In various embodiments, locking screw 750 may have an outside circumferential surface including a male thread pattern 751 at a distal end thereof, for example. Locking screw 750 may be disposed in screw guide aperture 731 and move forward and backward in the proximal / distal directions upon rotation of the locking screw 750. For example, locking screw 750 may include an internal circumferential surface 752 having any suitable size and shape for engaging with a driver to rotate locking screw 750. For example, a hexolobular shape, a torx shape, a hex shape, polygonal shape, etc. In various embodiments, the locking screw 750 may include a central aperture 753 extending therethrough; although, in some embodiments a distal end of locking screw 750 may be closed and the proximal side of locking screw 750 may still have a central aperture 753 extending partially through locking screw 750. In at least one embodiment, a distal end of locking screw 750 is closed and an outside distal surface of locking screw 750 may have a hemispherical and/or cup like shape that is indented or outdented for applying a compressive force at a point location. In other embodiments, a distal surface of locking screw 750 may be substantially flat and/or planar for applying a relatively more distributed compressive force. In the example embodiment, locking screw 750 may include a head portion 754, comprising an annular ring that extends out laterally farther than the maximum diameter of the threads of thread pattern 751, for example. For example, a diameter of head portion 754 may be larger than a maximum diameter of thread pattern 751, for example. However, in other embodiments, a diameter of head portion 754 may be about the same and/or substantially the same as a maximum diameter of thread pattern 751. In the example embodiment, locking screw 750 may include a smooth shaft portion 55 disposed central to and between thread pattern 751 and head portion 754, for example. This may allow the locking screw 750 to move forward and backward within screw guide aperture 731 a distance before a distal surface of head portion 754 engages with corresponding surfaces of the superior and inferior endplates 710, 720, as will be explained in further detail below.

In various embodiments, locking screw 750 may fix a relative angle of inclination between the superior and inferior endplates 710, 720 (a lordotic angle). For example, locking screw 750 may be rotated such that it linearly translates and/or moves from a proximal end 700p towards the distal end 700d thereby urging various contact surfaces of the superior endplate 710, core 730, and inferior endplate 720 into frictional engagement. For example still, locking screw 750 may apply a compressive force frictionally engaging the superior endplate 710, core 730, and inferior endplate 720 such that they are locked in a relative position to one another, as will be explained in further detail below.

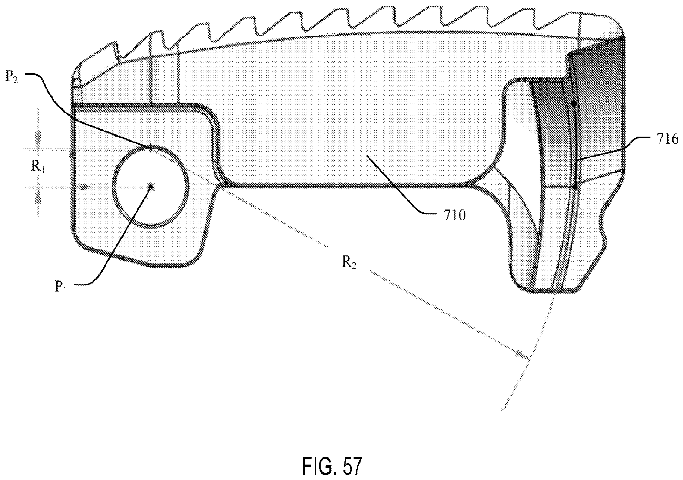

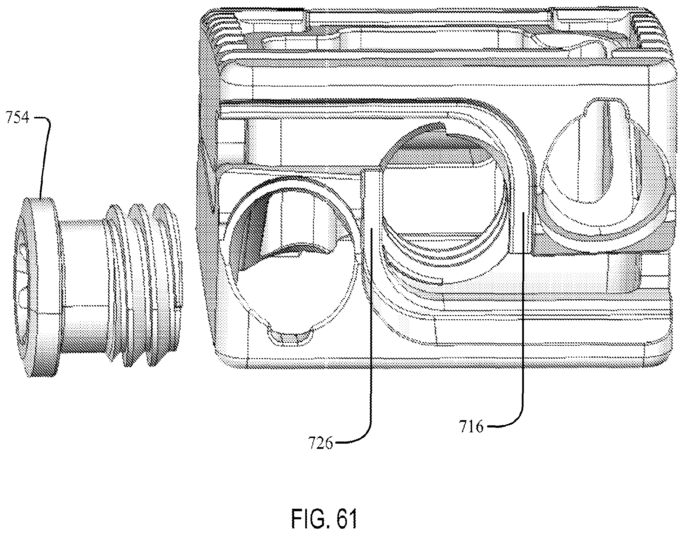

FIG. 57 illustrates a side view of superior endplate 710. In the example embodiment, and as explained above, superior endplate 710 may include a pair of pin receiving apertures 712a, 712b. In the side view of FIG. 57, only pin receiving aperture 712b is labeled. In various embodiments, pin receiving apertures 712a, 712b may be coaxially aligned circular apertures having the same radius, for example. As illustrated, pin receiving aperture 712b may comprise a circle having a radius R₁ and a center point P₁ defining a center of pin receiving apertures 712a, 712b and/or an axis of rotation that superior endplate 710 may rotate and/or pivot with respect to. For example, superior endplate 710 may be hingedly coupled to pin 740 and rotatable about an axis of rotation defined by center point P₁, for example. Additionally, in the example embodiment, superior endplate 710 may include an engagement surface 716 (see also FIG. 61). In various embodiments, engagement surface 716 may be a curved surface defined (in part or in total) by a segment of a circle having a radius R₂. In various embodiments, a center point P₂ of a circle defining the curved engagement surface 716 may be offset from center point P₁, for example. In the example embodiment, center point P₂ is vertically above center point P₁ by a distance approximating radius R₁. However, in other embodiments, center point P₂ may be offset by a greater amount or even a lesser amount than illustrated. In various embodiments, R₁ may be about 0.5mm to about 1 mm and R₂ may be about 7 mm to about 12 mm. In at least one embodiment R₁ is about 0.75 mm and R₂ is about 9.25 mm. In various embodiments, the inferior endplate 720 may also have a similar geometrical relationship.

Consistent with the disclosure herein, a geometrical relationship between the offset center points P₁ and P₂ may have several advantages in terms of operability and functionality. At least one advantage is that the superior endplate 710 may have a natural tendency to apply a force against the head portion 754 of locking screw 750 such that locking screw 750 may function similar to a wedge preventing implant 700 from fully collapsing. For example, in various embodiments, a superior vertebrae and an inferior vertebrae may apply a closing force against implant 700 and the offset radii arrangement as explained above may facilitate the engagement surface 716 naturally contacting head portion 754 of locking screw 750. For example still, an end user such as a surgeon may expand implant 700 and the offset arrangement explained above may facilitate the function of keeping implant 700 lordosed at the chosen angle.

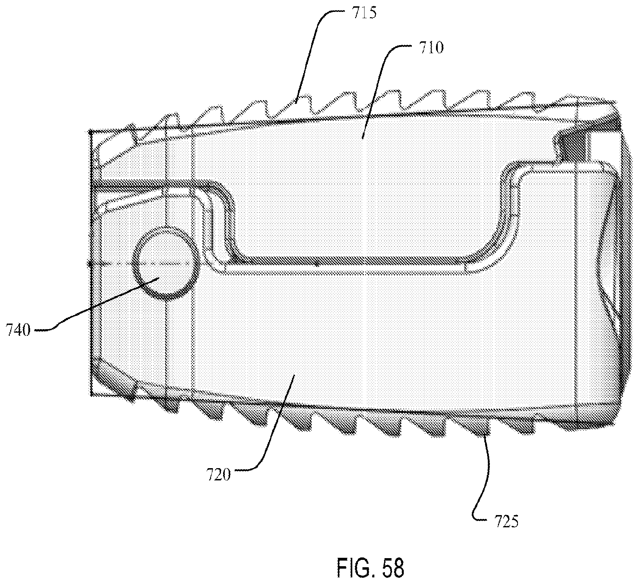

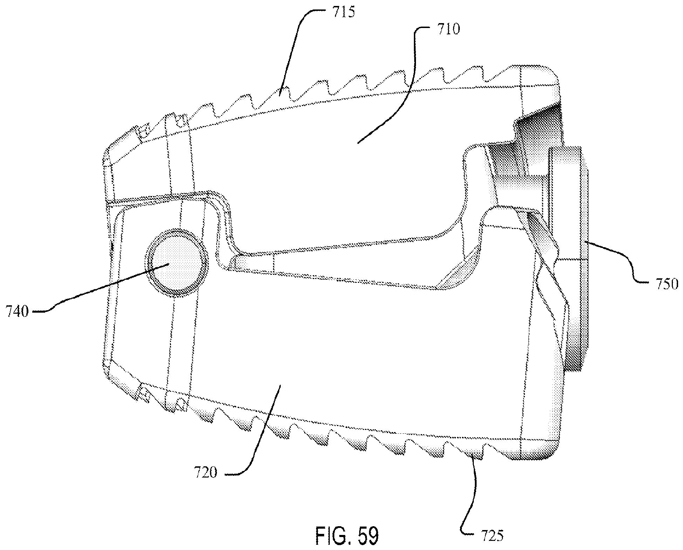

FIG. 58 is a side view of implant 700 in a collapsed position. In the illustrated embodiment, it is shown that superior endplate 710 includes a plurality of engagement features 15 and the inferior endplate 720 includes a plurality of engagement features 25. In the example embodiment, engagement features 715, 725 may comprise teeth or ridges extending in a lateral direction across the exposed surfaces of implant 700, for example. FIG. 59 is a side view of implant 700 in an expanded position. In the expanded position, it is shown that superior endplate 710 and inferior endplate 720 are inclined with respect to one another while core 730 retains locking screw 750 therein. In the expanded position, the superior endplate 710 may have pivoted about pin 740 upwards in the vertical direction and the inferior endplate 720 may have pivoted about pin 740 downwards in the vertical direction such that implant 700 is lordosed.

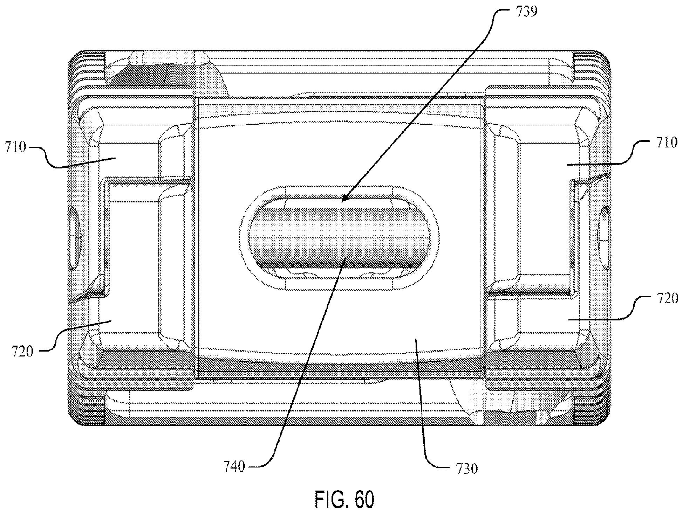

FIG. 60 is a rear view of implant 700. In the example embodiment, it is shown that pin 740 extends through a slotted aperture 739 of core 730. In the example embodiment, slotted aperture 739 may extend in a lateral direction on a distal surface of core 730 and include curved ends at opposite lateral sides thereof. Additionally, a void space is shown surrounding pin 740 which may facilitate boney ingrowth during a fusion process, for example. FIG. 60 also illustrates that the superior endplate 710 and inferior endplate 720 are pivotally mated together.

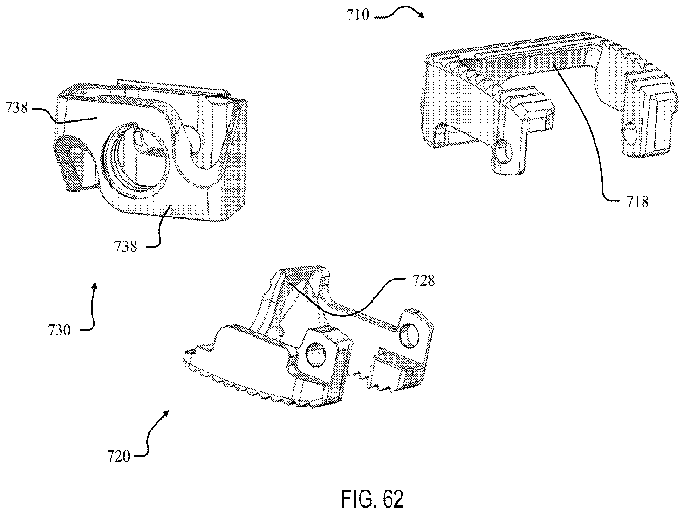

FIG. 61 illustrates a front view of implant 700 and a side view of locking screw 750. In the example illustration, it is shown that a backside (distal side) of head portion 754 may contact screw engagement surface 716 of superior endplate 710 and screw engagement surface 726 of inferior endplate 720. Screw engagement surfaces 716, 726 may comprise curved indentations having a profile that corresponds to a radius of curvature of head portion 754. For example, screw engagement surfaces 716, 726 may be shaped in a similar way to one another and locking screw 750 to contact the backside (distal side) of head portion 754 of locking screw 750 while also having enough clearance laterally for locking screw 750 to rotate. In the example embodiment, when locking screw 750 is sufficiently tightened the backside (distal side) of head portion 754 may push against screw engagement surfaces 716, 726 causing various internal surfaces of superior endplate 710 and inferior endplate 720 to frictionally engage and/or bind together as will be explained with reference to FIG. 62 below.

FIG. 62 is an exploded parts view with the superior endplate 710, inferior endplate 720, and core 730 rotated to illustrate the various surfaces that frictionally engage and/or bind together in a locked position. For example, superior endplate 710 may include a first binding surface 718 that may engage with and/or frictionally bind with a corresponding portion of binding surface 738 of core 730. First binding surface 718 may extend laterally on an upper interior surface of superior endplate 710, for example. Similarly, inferior endplate 720 may include a second binding surface 728 that may bind with and/or frictionally engage with a corresponding portion of binding surface 738 of core 730. In various embodiments, binding surfaces may be referred to as high friction surfaces, and/or engagement surfaces. In various embodiments, binding surface 738 of core 730 may face a proximal direction, and binding surfaces 718 and 728 may face a distal direction. In at least one embodiment, binding surfaces 718, 728, 738 comprise a high friction, roughened, and/or textured surface to facilitate jamming. In various embodiments, binding surfaces 718, 728, 738 may be surface roughened by a grit blast process. For example, an abrasive grit blasting process such as a sandblasting process including a surface treatment process to roughen the corresponding treated surfaces.

Accordingly, when locking screw 750 is sufficiently tightened the head portion 754 may push against screw engagement surfaces 716, 726 of superior endplate 710 and inferior endplate 720, respectively, thereby urging binding surfaces 718, 728, and 738 into a high friction and direct contact arrangement. In various embodiments, this high friction arrangement is sufficient to withstand a closing compressive force between a superior vertebra and an inferior vertebra. Additionally, locking screw 750 may function as a wedge between the curved engagement surfaces 716, 726 further preventing the collapse of implant 700. As explained herein, embodiments in accordance with the principles of this disclosure provide a highly adjustable implant 700 having an optimized and/or increased interior void space to facilitate a fusion process. In various example embodiments, implant 700 may be formed solely of five components, superior endplate 710, inferior endplate 720, core 730, pin 740, and locking screw 750. However, other embodiments may have more or less components and the aforementioned listing of components is not necessarily a precise and/or required listing.

In operation, a surgeon may expand implant 700 using an expansion tool. For example, an expansion tool having corresponding end portions that engage with gripping indentations 719, 729 and force open implant 700. Thereafter, and before fully tightening locking screw 750, implant 700 may naturally be biased towards a collapsed position as explained above yet may be prevented from collapsing due to engagement surfaces 716 and 726 and locking screw 750. For example, at least one of engagement surfaces 716, 726 may comprise a curved surface defined by a segment of a circle having a center point that is offset with respect to a center point and/or axis of extension of pin 740. Thereafter, an end user may tighten locking screw 750 such that locking screw 750 applies a compressive force against engagement surfaces 716 and 726 thereby pushing the superior endplate 710 and inferior endplate 720 against core 730. For example, locking screw 750 may apply a compressive force pushing the binding surfaces 718, 728 into a high friction engagement relationship with binding surface 738. As used herein, the term compressive force does not necessarily require that mechanical deflection occur but rather that two objects are pushed into direct contact by an applied force.

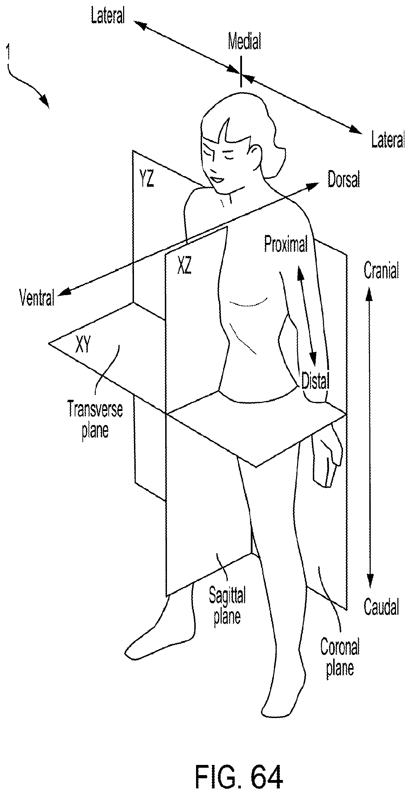

FIG. 63 is a reference drawing showing the human spine of which various disclosed implant embodiments may be installed in. FIG. 64 is a reference drawing showing various planes and reference directions of which the various disclosed implant embodiments may move in or act in with reference to a patient 1.

It should be understood that various aspects disclosed herein may be combined in different combinations than the combinations specifically presented in the description and accompanying drawings. For example, features, functionality, and components from one embodiment may be combined with another embodiment and vice versa unless the context clearly indicates otherwise. Similarly, features, functionality, and components may be omitted unless the context clearly indicates otherwise. It should also be understood that, depending on the example, certain acts or events of any of the processes or methods (not claimed) described herein may be performed in a different sequence, may be added, merged, or left out altogether (e.g., all described acts or events may not be necessary to carry out the techniques).

Unless otherwise specifically defined herein, all terms are to be given their broadest possible interpretation including meanings implied from the specification as well as meanings understood by those skilled in the art and/or as defined in dictionaries, treatises, etc. It must also be noted that, as used in the specification and the appended claims, the singular forms "a," "an" and "the" include plural referents unless otherwise specified, and that the terms "comprises" and/ or "comprising," when used in this specification, specify the presence of stated features, elements, and/or components, but do not preclude the presence or addition of one or more other features, steps, operations, elements, components, and/or groups thereof.

## Claims

1. An expandable implant (100) movable between a contracted position and an expanded position, comprising:
an expandable body extending from a proximal end (100P) to a distal end (100D) in a proximal-to-distal direction and extending from a first lateral side (100L) to a second lateral side (100L) in a widthwise direction, the expandable body being defined by a superior endplate (10) and an inferior endplate (20) that are hingedly connected;
the superior endplate (10) comprising a first core (15) having a screw slot (15a) and a distal engagement surface (15d);
the inferior endplate (20) comprising a second core (25) having a proximal engagement surface (25p); and
a locking screw (50) extendable through the first core (15) and second core (25) and movable between a locked position and an unlocked position,
wherein, in the locked position, the locking screw (50) urges the distal engagement surface (15d) of the first core (15) against the proximal engagement surface (25p) of the second core (25),
wherein:
the superior endplate (10) further comprises a channel (12) located adjacent the distal end (100D) and extending in the widthwise direction; and
the inferior endplate (20) further comprises a rail (40) located adjacent the distal end (100D) and extending in the widthwise direction, the rail (40) having a size and shape generally corresponding to a size and shape of the channel (12) and being located within the channel (12),
**characterized in that**
the superior endplate (10) comprises two hook portions (12a, 12b) adjacent the channel (12);
the inferior endplate (20) comprises two relief portions (40a, 40b) adjacent the rail (40);
each of the two hook portions (12a, 12b) has a size and shape generally corresponding to a size and shape of one of the two relief portions (40a, 40b), respectively;
each of the two hook portions (12a, 12b) is disposed within one of the two relief portions (40a, 40b), respectively,
the superior endplate (10) comprises a first plurality of engagement features (14) that are angled about 20 degrees to about 40 degrees with respect to a proximal surface of the superior endplate (10); and
the inferior endplate (20) comprises a second plurality of engagement features (14) that are angled about 20 degrees to about 40 degrees with respect to a proximal surface of the inferior endplate (20).

2. The expandable implant (100) of claim 1, wherein:
the superior endplate (10) comprises a first gripping indentation (419Z) located at a proximal end thereof; and
the inferior endplate (20) comprises a second gripping indentation (419Z) located at a proximal end thereof,
wherein optionally:
the first gripping indentation (419Z) comprises a slot having a superior curved surface (419S) and an inferior curved surface (419I); and
the second gripping indentation (419Z) comprises a slot having a superior curved surface (419S) and an inferior curved surface (419I).

3. The expandable implant (100) of claim 1, wherein:
the distal engagement surface (15d) of the first core (15) comprises a first curved surface;
the proximal engagement surface (25p) of the second core (25) comprises a second curved surface; and
the first curved surface is defined by a radius of a circle having a center point that is offset from an axis of rotation of the rail (40).

4. The expandable implant (100) of claim 1,
wherein in a cross-section view, the channel (12) comprises an arcuate shape and the rail (40) comprises an arcuate shape.

5. The expandable implant (100) of claim 1, wherein:
the locking screw (50) comprises a threaded end (51) and a drive feature (53a, 53b) separated by a fracture surface (55) for separating the locking screw (50);
the first core (15) is disposed proximally with respect to the second core (25); and
the locking screw (50) is threadably engaged with at least one of the first core (15) and second core (25).

6. The expandable implant (100) of claim 1, wherein:
the locking screw (50) extends in a proximal to distal direction along a longitudinal axis and is configured to be broken into a proximal portion and a distal portion at a fracture surface (55); and
when broken, the fracture surface (55) is recessed relative to a sidewall of the locking screw (50).

7. A system including a medical implant and a surgical tool, the system comprising:
an expandable implant (100) according to claim 1; and
the surgical tool (300) for moving the expandable implant (100) from a contracted position to an expanded position and for moving the locking screw (50) between the locked position and the unlocked position, the surgical tool (300) being capable of moving the locking screw (50) into the locked position while supporting the expandable implant (100) in the expanded position.

8. The system of claim 7, wherein:
the superior endplate (10) comprises a first gripping indentation (419Z) located at a proximal end thereof;
the inferior endplate (20) comprises a second gripping indentation (419Z) located at a proximal end thereof;
the surgical tool (300) comprises a first gripping protrusion (619) having a size and shape generally corresponding to a size and shape of the first gripping indentation (419Z); and
the surgical tool comprises a second gripping protrusion (629) having a size and shape generally corresponding to a size and shape of the second gripping indentation (419Z),
wherein optionally:
the first gripping indentation (419Z) comprises a slot having a superior curved surface (419S) and an inferior curved surface (419I); and
the second gripping indentation (419Z) comprises a slot having a superior curved surface (419S) and an inferior curved surface (419I).

9. The system of claim 7, wherein the surgical tool comprises a pivotal linkage assembly (608) including a first arm (610) and a second arm (611),
wherein, optionally, the surgical tool (300) comprises a body portion (607), a handle (601), and a third arm (620), the third arm (620) being fixed relative to the body portion (607).

10. The system of claim 7, wherein the surgical tool (300) further comprises:
a body portion (607) having an aperture (607A) extending therethrough;
a pivotal linkage assembly (608) including a first arm (611) and a second arm (612);
a third arm (620) that is fixed relative to the body portion (607);
an outer shaft (602) extending through the body portion (607) and having a drive feature (602A) at a distal end thereof for rotating the locking screw (50) from the unlocked position to the locked position; and
an inner shaft (606) extending through the outer shaft and having a first thread pattern (606T) at an end thereof for drawing the expandable implant (100) towards the surgical tool (300).

11. The system of claim 10, wherein:
the locking screw (50) comprises a recessed fracture surface (55) for separating the locking screw (50) into a proximal portion and a distal portion,
the surgical tool (300) is configured to separate the locking screw (50) into the proximal portion and distal portion while the expandable implant (100) is in the expanded position and retain the proximal portion of the locking screw (50) via the inner shaft.

12. The system of claim 10, wherein the surgical tool (300) further comprises an actuator (605) for causing the pivotal linkage assembly (608) to pivot relative to the third arm (620) and thereby expand the expandable implant (100).

## Patentansprüche

1. Expandierbares Implantat (100), das zwischen einer zusammengezogenen Position und einer expandierten Position bewegbar ist, umfassend:
einen expandierbaren Körper, der sich von einem proximalen Ende (100P) zu einem distalen Ende (100D) in einer Richtung von proximal nach distal erstreckt und sich von einer ersten lateralen Seite (100L) zu einer zweiten lateralen Seite (100L) in einer Breitenrichtung erstreckt, wobei der expandierbare Körper durch eine obere Endplatte (10) und eine untere Endplatte (20) definiert ist, die gelenkig verbunden sind;
die obere Endplatte (10) umfassend einen ersten Kern (15), der einen Schraubenschlitz (15a) und eine distale Eingriffsoberfläche (15d) aufweist;
die untere Endplatte (20) umfassend einen zweiten Kern (25), der eine proximale Eingriffsoberfläche (25p) aufweist; und
eine Verriegelungsschraube (50), die durch den ersten Kern (15) und den zweiten Kern (25) hindurch erstreckbar und zwischen einer verriegelten Position und einer entriegelten Position bewegbar ist,
wobei in der verriegelten Position die Verriegelungsschraube (50) die distale Eingriffsoberfläche (15d) des ersten Kerns (15) gegen die proximale Eingriffsoberfläche (25p) des zweiten Kerns (25) drückt, wobei:
die obere Endplatte (10) ferner einen Kanal (12) umfasst, der sich angrenzend an das distale Ende (100D) befindet und sich in der Breitenrichtung erstreckt; und
die untere Endplatte (20) ferner eine Schiene (40) umfasst, die sich angrenzend an das distale Ende (100D) befindet und sich in der Breitenrichtung erstreckt, wobei die Schiene (40) eine Größe und eine Form aufweist, die im Allgemeinen einer Größe und einer Form des Kanals (12) entsprechen, und sich innerhalb des Kanals (12) befindet,
**dadurch gekennzeichnet, dass**
die obere Endplatte (10) zwei Hakenabschnitte (12a, 12b) angrenzend an den Kanal (12) umfasst;
die untere Endplatte (20) zwei Aussparungsabschnitte (40a, 40b) angrenzend an die Schiene (40) umfasst;
jeder der zwei Hakenabschnitte (12a, 12b) eine Größe und eine Form aufweist, die im Allgemeinen jeweils einer Größe und einer Form eines der zwei Aussparungsabschnitte (40a, 40b) entsprechen;
jeder der zwei Hakenabschnitte (12a, 12b) jeweils innerhalb eines der zwei Aussparungsabschnitte (40a, 40b) angeordnet ist,
die obere Endplatte (10) eine erste Vielzahl von Eingriffsmerkmalen (14) umfasst, die um etwa 20 Grad bis etwa 40 Grad hinsichtlich einer proximalen Oberfläche der oberen Endplatte (10) abgewinkelt ist; und
die untere Endplatte (20) eine zweite Vielzahl von Eingriffsmerkmalen (14) umfasst, die um etwa 20 Grad bis etwa 40 Grad hinsichtlich einer proximalen Oberfläche der unteren Endplatte (20) abgewinkelt ist.

2. Expandierbares Implantat (100) nach Anspruch 1, wobei:
die obere Endplatte (10) eine erste Greifkerbe (419Z) umfasst, die sich an einem proximalen Ende davon befindet; und
die untere Endplatte (20) eine zweite Greifkerbe (419Z) umfasst, die sich an einem proximalen Ende davon befindet,
wobei optional:
die erste Greifkerbe (419Z) einen Schlitz umfasst, der eine obere gekrümmte Oberfläche (419S) und eine untere gekrümmte Oberfläche (419I) aufweist; und
die zweite Greifkerbe (419Z) einen Schlitz umfasst, der eine obere gekrümmte Oberfläche (419S) und eine untere gekrümmte Oberfläche (419I) aufweist.

3. Expandierbares Implantat (100) nach Anspruch 1, wobei:
die distale Eingriffsoberfläche (15d) des ersten Kerns (15) eine erste gekrümmte Oberfläche umfasst;
die proximale Eingriffsoberfläche (25p) des zweiten Kerns (25) eine zweite gekrümmte Oberfläche umfasst; und
die erste gekrümmte Oberfläche durch einen Radius eines Kreises definiert ist, der eine Zentrierspitze aufweist, die von einer Drehachse der Schiene (40) versetzt ist.

4. Expandierbares Implantat (100) nach Anspruch 1,
wobei in einer Querschnittsansicht der Kanal (12) eine bogenförmige Form umfasst und die Schiene (40) eine bogenförmige Form umfasst.

5. Expandierbares Implantat (100) nach Anspruch 1, wobei:
die Verriegelungsschraube (50) ein Gewindeende (51) und ein Antriebsmerkmal (53a, 53b) umfasst, die durch eine Bruchoberfläche (55) zum Trennen der Verriegelungsschraube (50) getrennt sind;
der erste Kern (15) proximal hinsichtlich des zweiten Kerns (25) angeordnet ist; und
die Verriegelungsschraube (50) mit mindestens einem von dem ersten Kern (15) und dem zweiten Kern (25) in Gewindeeingriff steht.

6. Expandierbares Implantat (100) nach Anspruch 1, wobei:
die Verriegelungsschraube (50) sich in einer Richtung von proximal nach distal entlang einer Längsachse erstreckt und konfiguriert ist, um an einer Bruchoberfläche (55) in einen proximalen Abschnitt und einen distalen Abschnitt gebrochen zu werden; und
wenn gebrochen, die Bruchoberfläche (55) relativ zu einer Seitenwand der Verriegelungsschraube (50) zurückgesetzt ist.

7. System, das ein medizinisches Implantat und ein chirurgisches Werkzeug einschließt, das System umfassend:
ein expandierbares Implantat (100) nach Anspruch 1; und
das chirurgische Werkzeug (300) zum Bewegen des expandierbaren Implantats (100) von einer kontrahierten Position in eine expandierte Position und zum Bewegen der Verriegelungsschraube (50) zwischen der verriegelten Position und der entriegelten Position, wobei das chirurgische Werkzeug (300) in der Lage ist, die Verriegelungsschraube (50) in die verriegelte Position zu bewegen, während es das expandierbare Implantat (100) in der expandierten Position stützt.

8. System nach Anspruch 7, wobei:
die obere Endplatte (10) eine erste Greifkerbe (419Z) umfasst, die sich an einem proximalen Ende davon befindet;
die untere Endplatte (20) eine zweite Greifkerbe (419Z) umfasst, die sich an einem proximalen Ende davon befindet;
das chirurgische Werkzeug (300) einen ersten Greifvorsprung (619) umfasst, der eine Größe und eine Form aufweist, die im Allgemeinen einer Größe und einer Form der ersten Greifkerbe (419Z) entsprechen; und
das chirurgische Werkzeug einen zweiten Greifvorsprung (629) umfasst, der eine Größe und eine Form aufweist, die im Allgemeinen einer Größe und einer Form der zweiten Greifkerbe (419Z) entsprechen,
wobei optional:
die erste Greifkerbe (419Z) einen Schlitz umfasst, der eine obere gekrümmte Oberfläche (419S) und eine untere gekrümmte Oberfläche (419I) aufweist; und
die zweite Greifkerbe (419Z) einen Schlitz umfasst, der eine obere gekrümmte Oberfläche (419S) und eine untere gekrümmte Oberfläche (419I) aufweist.

9. System nach Anspruch 7, wobei das chirurgische Werkzeug eine schwenkbare Gelenkanordnung (608) umfasst, die einen ersten Arm (610) und einen zweiten Arm (611) einschließt,
wobei, optional, das chirurgische Werkzeug (300) einen Körperabschnitt (607), einen Griff (601) und einen dritten Arm (620) umfasst, wobei der dritte Arm (620) relativ zu dem Körperabschnitt (607) fixiert ist.

10. System nach Anspruch 7, wobei das chirurgische Werkzeug (300) ferner umfasst:
einen Körperabschnitt (607), der eine Öffnung (607A) aufweist, die sich dorthindurch erstreckt;
eine schwenkbare Gelenkanordnung (608), die einen ersten Arm (611) und einen zweiten Arm (612) einschließt;
einen dritten Arm (620), der relativ zu dem Körperabschnitt (607) fixiert ist;
einen äußeren Schaft (602), der sich durch den Körperabschnitt (607) hindurch erstreckt und an einem distalen Ende davon ein Antriebsmerkmal (602A) zum Drehen der Verriegelungsschraube (50) von der entriegelten Position in die verriegelte Position aufweist; und
einen inneren Schaft (606), der sich durch den äußeren Schaft hindurch erstreckt und an einem Ende davon ein erstes Gewindemuster (606T) zum Ziehen des expandierbaren Implantats (100) zu dem chirurgischen Werkzeug (300) hin aufweist.

11. System nach Anspruch 10, wobei:
die Verriegelungsschraube (50) eine vertiefte Bruchoberfläche (55) zum Trennen der Verriegelungsschraube (50) in einen proximalen Abschnitt und einen distalen Abschnitt umfasst,
das chirurgische Werkzeug (300) konfiguriert ist, um die Verriegelungsschraube (50) in den proximalen Abschnitt und den distalen Abschnitt zu trennen, während das expandierbare Implantat (100) in der expandierten Position ist, und den proximalen Abschnitt der Verriegelungsschraube (50) über den inneren Schaft zu halten.

12. System nach Anspruch 10, wobei das chirurgische Werkzeug (300) ferner einen Aktuator (605) zum Veranlassen der schwenkbaren Gelenkanordnung (608) umfasst, relativ zu dem dritten Arm (620) zu schwenken und dadurch das expandierbare Implantat (100) zu expandieren.

## Revendications

1. Implant expansible (100) pouvant être déplacé entre une position contractée et une position expansée, comprenant :
un corps expansible s'étendant d'une extrémité proximale (100P) à une extrémité distale (100D) dans une direction proximale à distale et s'étendant d'un premier côté latéral (100L) à un second côté latéral (100L) dans une direction de la largeur, le corps expansible étant défini par une plaque d'extrémité supérieure (10) et une plaque d'extrémité inférieure (20) qui sont reliées de manière articulée ;
la plaque d'extrémité supérieure (10) comprenant un premier noyau (15) ayant une fente de vis (15a) et une surface de mise en prise distale (15d) ;
la plaque d'extrémité inférieure (20) comprenant un second noyau (25) ayant une surface de mise en prise proximale (25p) ; et
une vis de blocage (50) pouvant s'étendre à travers le premier noyau (15) et le second noyau (25) et pouvant être déplacée entre une position verrouillée et une position déverrouillée,
dans lequel, en position verrouillée, la vis de blocage (50) pousse la surface de mise en prise distale (15d) du premier noyau (15) contre la surface de mise en prise proximale (25p) du second noyau (25), dans lequel :
la plaque d'extrémité supérieure (10) comprend en outre un canal (12) situé à côté de l'extrémité distale (100D) et s'étendant dans la direction de la largeur ; et
la plaque d'extrémité inférieure (20) comprend en outre un rail (40) situé à côté de l'extrémité distale (100D) et s'étendant dans la direction de la largeur, le rail (40) ayant une taille et une forme correspondant généralement à une taille et à une forme du canal (12) et étant situé à l'intérieur du canal (12),
**caractérisé en ce que**
la plaque d'extrémité supérieure (10) comprend deux parties de crochet (12a, 12b) adjacentes au canal (12) ;
la plaque d'extrémité inférieure (20) comprend deux parties en relief (40a, 40b) adjacentes au rail (40) ;
chacune des deux parties de crochet (12a, 12b) a une taille et une forme correspondant généralement à une taille et à une forme de l'une des deux parties en relief (40a, 40b), respectivement ;
chacune des deux parties de crochet (12a, 12b) est disposée à l'intérieur de l'une des deux parties en relief (40a, 40b), respectivement,
la plaque d'extrémité supérieure (10) comprend une première pluralité de caractéristiques de mise en prise (14) qui sont inclinées d'environ 20 degrés à environ 40 degrés par rapport à une surface proximale de la plaque d'extrémité supérieure (10) ; et
la plaque d'extrémité inférieure (20) comprend une seconde pluralité de caractéristiques de mise en prise (14) qui sont inclinées d'environ 20 degrés à environ 40 degrés par rapport à une surface proximale de la plaque d'extrémité inférieure (20).

2. Implant expansible (100) selon la revendication 1, dans lequel :
la plaque d'extrémité supérieure (10) comprend une première indentation de préhension (419Z) située au niveau d'une extrémité proximale de celle-ci ; et
la plaque d'extrémité inférieure (20) comprend une seconde indentation de préhension (419Z) située au niveau d'une extrémité proximale de celle-ci,
dans lequel, facultativement :
la première indentation de préhension (419Z) comprend une fente ayant une surface incurvée supérieure (419S) et une surface incurvée inférieure (419I) ; et
la seconde indentation de préhension (419Z) comprend une fente ayant une surface incurvée supérieure (419S) et une surface incurvée inférieure (419I).

3. Implant expansible (100) selon la revendication 1, dans lequel :
la surface de mise en prise distale (15d) du premier noyau (15) comprend une première surface incurvée ;
la surface de mise en prise proximale (25p) du second noyau (25) comprend une seconde surface incurvée ; et
la première surface incurvée est définie par un rayon d'un cercle ayant un point central qui est décalé par rapport à un axe de rotation du rail (40).

4. Implant expansible (100) selon la revendication 1,
dans lequel, dans une vue en coupe transversale, le canal (12) comprend une forme arquée et le rail (40) comprend une forme arquée.

5. Implant expansible (100) selon la revendication 1, dans lequel :
la vis de blocage (50) comprend une extrémité filetée (51) et une caractéristique d'entraînement (53a, 53b) séparés par une surface de fracture (55) permettant de séparer la vis de blocage (50) ;
le premier noyau (15) est disposé de manière proximale par rapport au second noyau (25) ; et
la vis de blocage (50) est en prise de manière filetée avec au moins l'un parmi le premier noyau (15) et le second noyau (25).

6. Implant expansible (100) selon la revendication 1, dans lequel :
la vis de blocage (50) s'étend dans une direction proximale à distale le long d'un axe longitudinal et est conçue pour être brisée en une partie proximale et une partie distale au niveau d'une surface de fracture (55) ; et
lorsqu'elle est rompue, la surface de fracture (55) est en retrait par rapport à une paroi latérale de la vis de blocage (50).

7. Système comportant un implant médical et un outil chirurgical, le système comprenant :
un implant expansible (100) selon la revendication 1 ; et
l'outil chirurgical (300) permettant de déplacer l'implant expansible (100) d'une position contractée à une position expansée et permettant de déplacer la vis de blocage (50) entre la position verrouillée et la position déverrouillée, l'outil chirurgical (300) étant capable de déplacer la vis de blocage (50) dans la position verrouillée tout en soutenant l'implant expansible (100) dans la position expansée.

8. Système selon la revendication 7, dans lequel :
la plaque d'extrémité supérieure (10) comprend une première indentation de préhension (419Z) située au niveau d'une extrémité proximale de celle-ci ;
la plaque d'extrémité inférieure (20) comprend une seconde indentation de préhension (419Z) située au niveau d'une extrémité proximale de celle-ci ;
l'outil chirurgical (300) comprend une première saillie de préhension (619) ayant une taille et une forme correspondant généralement à une taille et à une forme de la première indentation de préhension (419Z) ; et
l'outil chirurgical comprend une seconde saillie de préhension (629) ayant une taille et une forme correspondant généralement à une taille et à une forme de la seconde indentation de préhension (419Z),
dans lequel, facultativement :
la première indentation de préhension (419Z) comprend une fente ayant une surface incurvée supérieure (419S) et une surface incurvée inférieure (419I) ; et
la seconde indentation de préhension (419Z) comprend une fente ayant une surface incurvée supérieure (419S) et une surface incurvée inférieure (419I).

9. Système selon la revendication 7, dans lequel l'outil chirurgical comprend un ensemble de liaison pivotante (608) comportant un premier bras (610) et un deuxième bras (611),
dans lequel, facultativement, l'outil chirurgical (300) comprend une partie corps (607), une poignée (601), et un troisième bras (620), le troisième bras (620) étant fixe par rapport à la partie corps (607).

10. Système selon la revendication 7, dans lequel l'outil chirurgical (300) comprend en outre :
une partie corps (607) ayant une ouverture (607A) s'étendant à travers elle ;
un ensemble de liaison pivotante (608) comportant un premier bras (611) et un second bras (612) ;
un troisième bras (620) qui est fixe par rapport à la partie corps (607) ;
une tige externe (602) s'étendant à travers la partie corps (607) et ayant une caractéristique d'entraînement (602A) au niveau d'une extrémité distale de celle-ci permettant de faire tourner la vis de verrouillage (50) de la position déverrouillée à la position verrouillée ; et
une tige interne (606) s'étendant à travers la tige externe et ayant un premier motif de filetage (606T) au niveau d'une extrémité de celle-ci permettant d'attirer l'implant expansible (100) vers l'outil chirurgical (300).

11. Système selon la revendication 10, dans lequel :
la vis de blocage (50) comprend une surface de fracture en retrait (55) permettant de séparer la vis de blocage (50) en une partie proximale et une partie distale,
l'outil chirurgical (300) est conçu pour séparer la vis de verrouillage (50) en la partie proximale et la partie distale pendant que l'implant expansible (100) est en position expansée et retenir la partie proximale de la vis de verrouillage (50) par l'intermédiaire de la tige interne.

12. Système selon la revendication 10, dans lequel l'outil chirurgical (300) comprend en outre un actionneur (605) permettant d'amener l'ensemble de liaison pivotante (608) à pivoter par rapport au troisième bras (620) et ainsi d'expanser l'implant expansible (100).
